# EUROPEAN PATENT SPECIFICATION

(11) **EP 1 765 861 B1**
(45) Date of publication and mention of the grant of the patent: **15.02.2012**
(21) Application number: 05748535.1
(22) Date of filing: 17.06.2005
(51) Int. Cl.: C07K 14/705, C07K 16/28, A61K 38/17, A61P 25/28

(54) **FGFR BINDING PEPTIDES**
FGFR-BINDENDE PEPTIDE
PEPTIDES DE LIAISON A FGFR

(30) Priority: 18.06.2004 DK 200400963
(43) Date of publication of application: 28.03.2007
(73) Proprietor: Enkam Pharmaceuticals A/S, 2100 Copenhagen 0 (DK)
(72) Inventor: BOCK, Eisabeth, DK-2920 Charlottenlund (DK); BEREZIN, Vladimir, DK-2200 Copenhagen N (DK)
(74) Representative: Björk, Frida Magdalena
(86) International application number: PCT/DK2005/000400
(87) International publication number: WO 2005/123759

(56) References cited:
- WO-A-01/96364
- WO-A-03/016351
- WO-A-2005/049641
- US-A1- 2002 104 122

## Description

### Field of invention

The present invention relates to new peptide compounds derived from the sequence of the fibronectin type-III module 1 (F3, 1) of the neural cell adhesion molecule (NCAM) which are capable of binding to fibroblast growth factor receptor (FGFR). The invention also relates to pharmaceutical compositions comprising the compounds and uses thereof in treatment and/or prevention of different pathological conditions wherein FGFR and/or NCAM play a role in pathology and/or recovery from disease.

### Background of invention

Brain plasticity and the mechanisms controlling plasticity are central to learning and memory as well as the recovery of function after brain injury. While it is clear that neurotrophic factors are one of the molecular classes that continue to regulate brain plasticity in the adult central nervous system (CNS), less appreciated but equally profound is the role of cell adhesion molecules (CAMs) in plasticity mechanisms such as long term potentiation, preservation of neurons and regeneration. Ironically, however, CAMs can also reorganise the extra-cellular space and cause disturbances that drive the development of brain pathology in conditions such as Alzheimer's disease and multiple sclerosis. Candidate molecules include the amyloid precursor protein, which shares many properties of a classical CAM and beta-amyloid, which can masquerade as a pseudo CAM. Beta-Amyloid serves as a nidus for the formation of senile plaques in Alzheimer's disease and like CAMs provides an environment for organising neurotrophic factors and other CAMs. Inflammatory responses evolve in this environment and can initiate a vicious cycle of perpetuated neuronal damage that is medicated by microglia, complement and other factors (Cotman et al. (1998) Prog Neurobiol. 55:659-69).

Neural cell adhesion molecules (CAMs) of the immunoglobulin superfamily nucleate and maintain groups of cells at key sites during early development and in the adult. In addition to their adhesive properties, CAMs homophylic and heterophylic interactions can affect intracellular signalling. Their ability to influence developmental events, including cell migration, proliferation, and differentiation may therefore result both from their adhesive as well as their signalling properties.

The neural cell adhesion molecule, NCAM, was the first discovered neural CAM. Since the discovery NCAM has been intensively studied and now it is well characterised (Ronn et al. (2000) Int J Dev Neurosci 18:193-9) NCAM belongs to the immunoglobulin (Ig) superfamily. Its extracellular part consists of five Ig-like and two fibronectin type III (F3) modules. NCAM assists both the cell-cell and cell-substratum interactions. NCAM binds to various extracellular matrix components such as heparin/heparan sulfate, chondroitin sulfate proteoglycans, and different types of collagen. Cell-cell interactions are mostly assisted by the NCAM homophilic interaction. The different modules of NCAM have been shown to perform distinct functions. Thus, NCAM homophilic binding is believed now to depend on the first three Ig modules. The heparin binding sequence is localised to the Ig2 module. NCAM also binds to the neural cell adhesion molecule L1. This interaction is believed to take place between the fourth Ig module of NCAM and an oligomannosidic moiety expressed on L1. The two membrane-proximal F3 modules of NCAM have been shown involved in fibroblast growth factor receptor (FGFR) binding.

A number of research groups has now accumulated a large body of evidence indicating that intracellular signalling cascades underlying the NCAM-mediated axonal outgrowth are similar to signal transduction cascades which are activated due to stimulation of FGFR (Povlsen et al. (2003) Neurochem Res 1:127-41).

Fibroblast growth factor receptors (FGFRs) are the family of four closely related receptor protein tyrosine kinases consisting extracellularly of three Ig-like modules and intracellularly of a split tyrosine-kinase module (Powers et al. (2000) Endocr Relat Cancer 7:165-97). The receptors are known as key regulators of morphogenesis, development, angiogenesis, and wound healing. FGFR activation and signalling are dependent on dimerization of the receptor which is induced by a high affinity binding of its ligand, fibroblast growth factor (FGF), and it also requires participation of cell surface heparin or heparan sulphate proteoglycans. Fibroblast growth receptors play a prominent role in the functioning of the peripheral and central neural system (Jungnickel et al, (2004) Mol Cell Neurosci. 25:21-9; Reuss and von Bohlen und Halbach (2003) Cell Tissue Res. 313:139-57).

NCAM has been regarded as a member of a new class of putative alternative ligands of FGFR, and recently there has been obtained evidence for a direct interaction between NCAM and the receptor (Kiselyov et al. (2003) Structure (Camb) 11:691-701; WO 04/056865). The identified NCAM fragment which is directly involved in the interaction with FGFR has the sequence EVYVVAENQQGKSKA and derived from the second finronectin type-III (F3, 2) module of NCAM.

Recently, it has also been suggested that other fragments of NCAM may be involed in NCAM-FGFR interaction as well. This group of peptide fragments is derived from the first fibronectin type-III (F3,1) module of NCAM. Thus, WO 01/96364 has disclosed the 13 amino acid residues sequence SIDRVEPYSSTAQ, wherein the motif DRVE is claimed to be essential for stimulating neurite outgrowth and the motif PYSSTA to be responsible for the capability of the sequence to stimulate cell survival. The sequence and peptide fragments thereof comprising the latter motifs were suggested for treatment of diseases associated with neural cell loss due to trauma or disease and as medicaments for stimulating neural cell differentiation and survival. Lately, another patent application WO 05/014623 has described a 14 amino acid residues fragment of NCAM F31, 1 which has the sequence TIMGLKPETRYAVR. The sequence has been shown to be a neurite outgrowth stimulator. None of these two applications did demonstrate a direct binding of the sequences to FGFR and activating of the receptor.

### Summary of invention

The authors of the present invention demonstrates herein that the 13 amino acid residues sequence of WO 01/96364 extended to 17 amino acid residues, wherein 4 additional amino acid residues are added to the C-terminal of the sequence, is not any more capable of stimulating neurite outgrowth or neuronal cell survival, however, surprisingly, the 17 amino acid residues sequence is capable of stimulating learning and memory. Moreover, the 17 amino acid sequence is also capable of direct binding and activating FGFR. The authors of the present invention correlate these new features of the latter sequence with the presence of the additional C-terminal 4 amino acid residues which comprise an essential amino acid residue of the amino acid motif K/R-x^{p}-D/E/N/Q-x^{p}-x₀-S-x₁-x₂-x₃-D/E/N/Q-x^{p}, wherein x^{p} is any hydrophobic amino acid residue, x₀ is K, R or Y, and x₁, x₂, x₃ are indepently any amino acid residue. The authors of the present invention claims herein that this motif is essential for a capability of a peptide sequence to stimulate neural plasticity associated with learning and memory.

Thus, in the first aspect the invention relates to use of a contigous peptide of 11 to 18 amino acid residues compising the amino acid motif of formula (I)

K/R-x^{p}-D/E/N/Q-x^{p}-x₀-S-x₁-x₂-x₃-D/E/N/Q-x^{p}

wherein
X^{p} is independently selected from A, P, V or W,
x₀ is K, R or Y, and
x₁, x₂, x₃ are independently any amino acid residue.

A peptide comprising the motif may be used for the manufacturing of a medicament for stimulating learning and memory.

The motif of formula (I) is also important for a direct binding of a peptide to FGFR and activating the receptor, however, surprisingly, the authors of the present invention found out that the choice of a hydrophobic residue of the motif defines the array of biological activities that a peptide sequence comprising the motif has. Thus, the presence of proline (P) residue at any x^{p} position of the motif restricts capabilities of the sequence to the capability of stimulating learning and memory only, whereas any other hydrophobic residue at position x^{p} confers to the sequence both the capability of stimulating learning and memory, capability of stimulating neurite outgrowth and capability of stimulating neuronal cell survival. Accordingly, in another aspect the present invention relates to a compound comprising a contigous peptide sequence of 11 to 18 amino acid residues comprising the amino acid motif of the formula (II)

K/R-x^{p}-D/E/N/Q-x^{p}-x₀-S-x₁-x₂-x₃-D/E/N/Q-x^{p}

wherein
x^{p} is a hydrophobic amino acid residue independently selected from A, I, L, V, F or W,
x₀ is K, R or Y, and
x₁, x₂, x₃ are independently any amino acid residue

According to the invention a peptide comprising the latter motif is capable of stimulating neurite outgrowth, stimulating neural cell survival and stimulating neural plasticity associated with learning and memory and it is capable of binding and activating FGFR. Such sequence according to invention may advantageously be used for the manufacturing a medicament for treatment of a number of diseases wherein NCAM and/or FGFR play the major roles.

The invention relates also to pharmaceutical compositions and medicaments comprising peptides of the invention. Further, the invention features the compounds of formulae (I) or (II) for use in therapy comprising a step of administering a peptide sequence and/or pharmaceutical composition and/or medicament according to invention. Additionally, the invention relates to an antibody capable of recognising and binding to an epitope comprising an amino acid sequence of the formulas disclosed herein and use of said antibody for the modulating biological activity mediated by NCAM and/or FGFR.

### Description of Drawings

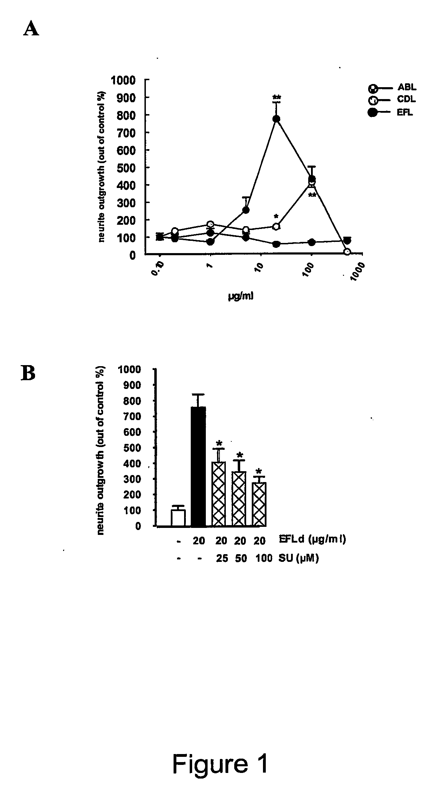

**Figure 1** **A** shows the effect of the CDL (SEQ ID NO: 1), ABL (SEQ ID NO: 2) and EFL (SEQ ID NO: 5) peptides on neurite outgrowth of rat cerebellar granular neurons (CGNs) in culture. The CDL and EFL peptides stimulate neurite outgrowth from CGNs. **B.** Neurite outgrowth stimulated by EFL is inhibited by SU5402, an inhibitor of fibroblast growth factor receptor 1 (FGFR1). Results from four independent experiments are in all cases expressed as a percentage ± SEM, with non-treated control cells set at 100%. *P<0.05, **P<0.01, ***P<0.001, compared with control. Paired student's t-test. For experiments, CGNs from 7-old-day rats were treated with the peptides for 24 hr. The cultures were fixed in 4% paraformaldehyde, followed by blocking with 1 % BSA and then immunostained with rabbit anti-rat GAP-43 primary antibodies, and then with secondary Alexa Fluor®488 goat anti-rabbit antibodies..

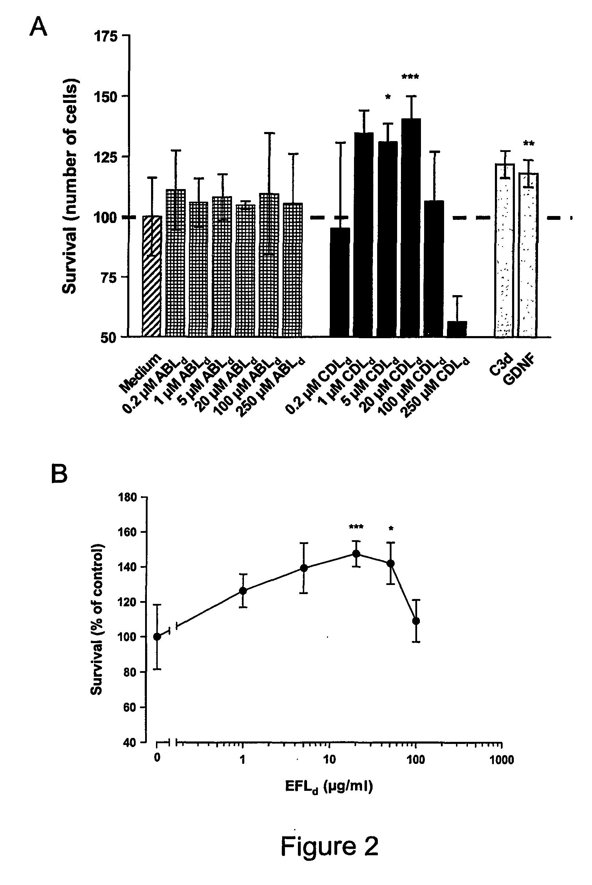

**Figure 2** **A** shows the effect of the dendrimeric forms of CDL (SEQ ID NO: 1) (CDLd) and ABL (SEQ ID NO: 2) (ABLd) peptides on survival of rat cerebellar granular neurons in culture. The effect of the peptides is compared to the effect of glia-derived neurotrophic factor (GDNF) and dendrimeric form of the C3 peptide (C3d). **B** demonstrates the effect of the EFL (SEQ ID NO: 5) dendrimer (EFLd) on on survival of rat cerebellar granular neurons in culture. *P<0.05, **P<0.01, ***P<0.001, compared with control. Paired student's t-test.

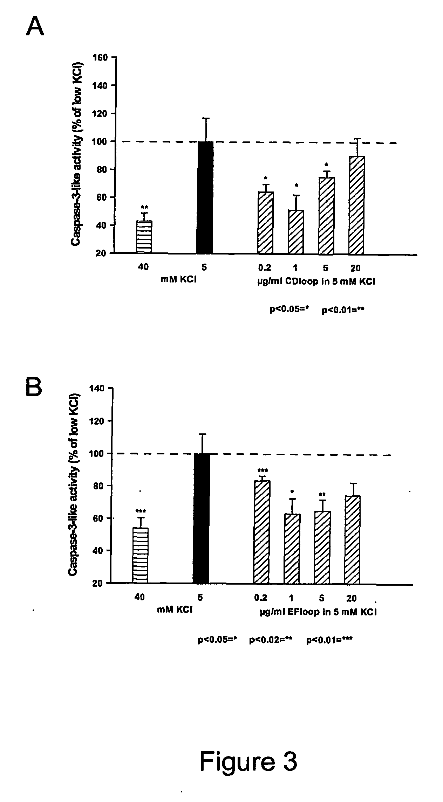

**Figure 3** shows the effect of the monomeric versions of CDL (SEQ ID NO: 1) (**A**) (CDloop) and EFL (SEQ ID NO: 5) (**B**) (EFloop) on caspase3-like activity in cultures of rat cerebellar granular neurons in low concentration of KCI (5mM) and high concentration of KCI (40 mM) in the medium. The effect of the peptide is compared to the effect of high concentration of KCl.. *P<0.05, **P<0.01, paired student's t-test

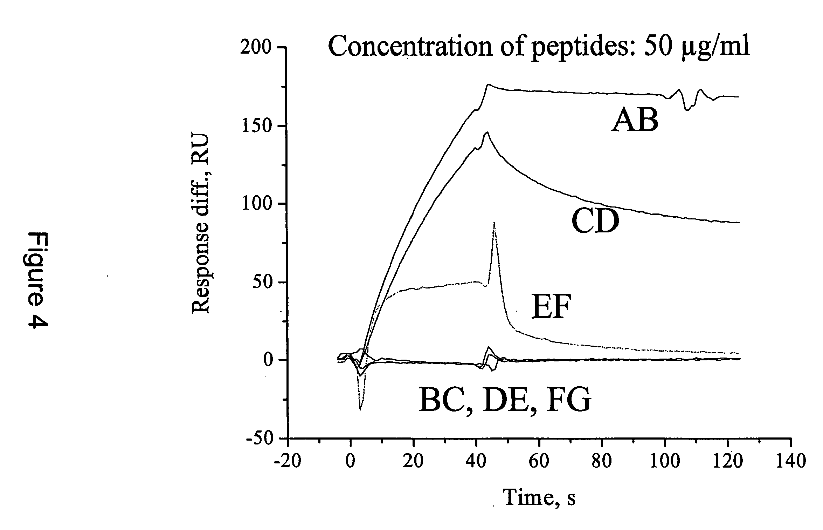

**Figure 4** demonstrates the binding of four different fragments of NCAM Fn-III,1 module having the "strand-loop-strand" structure to recombinant FGFR1 studied by means of surface plasmon resonance (SPR) analysis. The binding is given as a response difference (Resp. Diff.) between the binding to the sensor chip with the immobilized FGFR1 modules and a blank sensor chip (unspecific binding). Abbreviations: AB - A strand-loop-B strand (corresponds to the ABL peptide of SEQ ID NO: 2); CD - C strand-loop-D strand (corresponds to the CDL peptide of SEQ ID NO: 1); EF - E strand-loop-F strand (corresponds to the EFL peptide of SEQ ID NO: 5); BC - B strand-loop-C strand of F3, 1 module of NCAM; DE - D strand-loop-E strand of F3, 1 module of NCAM; FG - F strand-loop-G strand of F3, 1 module of NCAM.

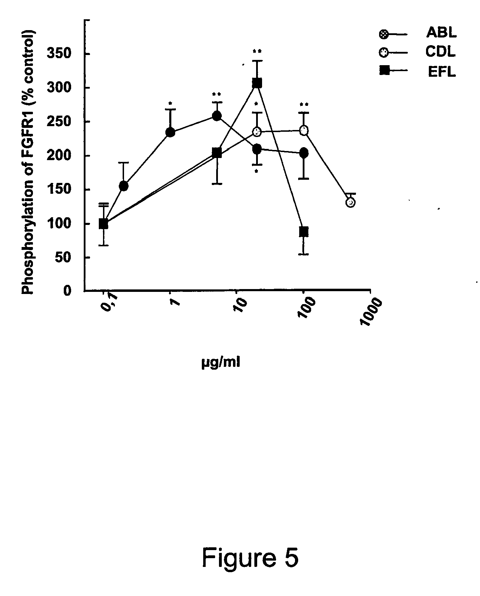

**Figure 5** demonstrates the results of quantificative analysis of phosphorylation of FGFR-1 by the ABL, CDL and EFL peptides. Results from at least four independent experiments are expressed as a percentage of FGFR1 phosphorylation ± SEM. For experiments, TREX-293 cells transfected with FGFR containing a C-terminal Strepll tag were stimulated with different concentrations of the peptides for 30 min. After stimulation, activated FGFR was immunopurified by anti-phosphtyrosine antibodies and then analyzed by western blotting by antibodies against the Strepll tag. The results from treated cells are compared with the resultas from non-treated cells (control, set to 100%). *P<0.05, **P<0.01, ***P<0.001, paired student's t-test.

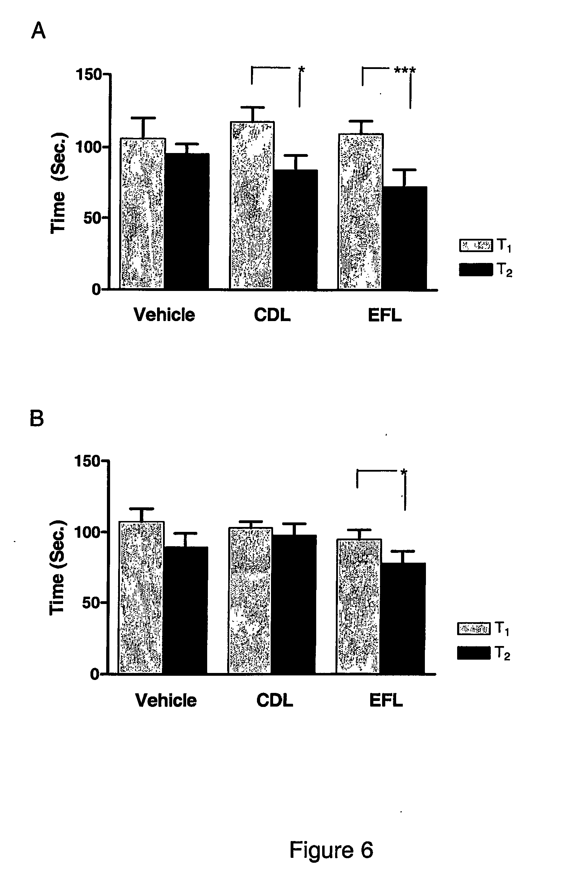

**Figure 6** shows the effect of administration of the CDL (Cdl), and EFL peptide in vivo on social behaviour of rats (the social recognition test): **A** Time spent with social investigation at 1st meeting (T₁) and second meeting (T₂) 1 h after peptide/vehicle administration. Results from 8-9 animals expressed as a percentage ±SEM. Paired t-test (*P<0.05 and ***P<0.001); **B** Time spent with social investigation at first meeting (T₁) and second meeting (T₂) 24h after peptide/vehicle administration. Results from 9 animals expressed as a percentage ±SEM. Paired t-test (*P<0.05).

### Detailed description of the invention

Compounds capable of modulating the function of fibroblast growth factor receptor (FGFR) and/or the neural cell adhesion molecule (NCAM) are of greatest importance in view of development of effective drugs for therapeutic treatment of a variety of diseases and pathologic conditions. Thus, it is an objection of the present invention to provide novel compounds capable of binding to FGFR and thereby activating FGFR.

### 1. Peptide sequences

In the present application the standard one-letter code for amino acid residues as well as the standard three-letter code are applied. Abbreviations for amino acids are in accordance with the recommendations in the IUPAC-IUB Joint Commission on Biochemical Nomenclature Eur. J. Biochem, 1984, vol. 184, pp 9-37. Throughout the description and claims either the three letter code or the one letter code for natural amino acids are used. Where the L or D form has not been specified it is to be understood that the amino acid in question has the natural L form, cf. Pure & Appl. Chem. Vol. (56(5) pp 595-624 (1984) or the D form, so that the peptides formed may be constituted of amino acids of L form, D form, or a sequence of mixed L forms and D forms.

Where nothing is specified it is to be understood that the C-terminal amino acid of a peptide of the invention exists as the free carboxylic acid, this may also be specified as "-OH". However, the C-terminal amino acid of a compound of the invention may be the amidated derivative, which is indicated as "-NH₂". Where nothing else is stated the N-terminal amino acid of a polypeptide comprise a free amino-group, this may also be specified as "H-".

Where nothing else is specified amino acid can be selected from any amino acid, whether naturally occurring or not, such as alfa amino acids, beta amino acids, and/or gamma amino acids. Accordingly, the group comprises but are not limited to: Ala, Val, Leu, Ile, Pro, Phe, Trp, Met, Gly, Ser, Thr, Cys, Tyr, Asn, Gln, Asp, Glu, Lys, Arg, His Aib, Nal, Sar, Orn, Lysine analogues, DAP, DAPA and 4Hyp.

Also, according to the invention modifications of the compounds/peptides may be performed, such as glycosylation and/or acetylation of the amino acids.

Basic amino acid residues are according to invention represented by the residues of amino acids Arg, Lys, and His, acidic amino acid residues - by the residues of amino acids Glu and Asp, hydrophobic amino acid residues - by the residues of amino acids Leu, Ile, Val, Phe, Trp, Pro and Ala, and polar uncharged amino acid residues - by the residues of amino acids Tyr, The, Ser, Gin and Asn.

In the further context of the description, the terms 'peptide' and peptide sequence might be used interchangeable. The scope of the present invention is defined by the claims.

In the first aspect the present invention provides a peptide of 11 to 18 amino acid residues compising the amino acid motif of formula (I)

K/R-x^{p}-D/E/N/Q-x^{p}-x₀-S-x₁-x₂-x₃-D/E/N/Q-x^{p}

wherein
x^{p} is independently selected from A, P, V or W,
x₀ is K, R or Y, and
x₁, x₂, x₃ are indepently any amino acid residue.

By the term "any hydrophobic residue" is meant a residue is selected from the residues of amino acids A, I, L, P, V, F or W.

Thus, in one embodiment the residue residue x^{p} may independently be selected from A, P, V or W. The term "independently" in the present context means that there are no any specific demands for the choice of an amino acid residue at any specified position.

Still, in another embodiment the x^{p} amino acid residue may be selected from A, V or W and the amino acid motif preferably is

K/R-V/A-D/E/N/Q-W-x₀-S-x₁-x₂-x₃-D/E/N/Q-V/A

wherein
x₀ is K, R or Y, and
x₁, x₂, x₃ are independently any amino acid residue.

In another embodiment the x^{p} amino acid residue may be selected from A, V or P the amino acid motif preferably is

K/R-V/A-D/E/N/Q-P-x₀-S-x₁-x₂-x₃-D/E/N/Q-V/A

wherein
x₀ is K, R or Y, and
x₁, x₂, x₃ are independently any amino acid residue.

According to the invention, the residues x₁, x₂, x₃ of any of the above motifs are not of major importance for biological activity of peptide sequences comprising the motifs of above and therefore said residues may independently be selected from any amino acid residues. However, the residues A, G, L, S, T, and E may in some embodiments be preferred.

Thus, a peptide the invention has a contiguous sequence of 11 to 18 amino acid residues comprising a motif as any of the motifs described above. The invention relates to a peptide which is an isolated peptide. This in the present context means that the peptide exists as an individual chemical entity and is used for the purposes of the invention as an individual chemical entity. The invention does not relate to peptides comprising the above motif(s), which are integrated parts of natural or recombinant polypeptides, such as for example the intergrated parts of polypeptides or fragments of the neural cell adhesion molecule (NCAM).

In one embodiment a peptide may have the length of 11 to 14 amino acid residues; in another embodiment the peptide may have the length of 15 to 18 amino acid residues. The length of a sequence such as 11, 12, 13, 14, 15, 16, 17 or 18 amino acid residues may be preferred depending on the embodiments.

The invention associates the structural features as described above with a capability of a peptide to bind and activate FGFR, to stimulate learning and memory, neurite outgrowth and /or neuronal cell survival.

The invention discloses herein particular peptide sequences which are capable of stimulating memory and learning, namely the sequences KAEWKSLGEEAWHSK (SEQ ID NO: 1), and SIDRVNPYSSTAQVQFD (SEQ ID NO: 3).

In some embodiments the sequence KAEWKSLGEEAWHSK (SEQ ID NO: 1) may be preferred, whereas in another embodiments the sequence SIDRVNPYSSTAQVQFD (SEQ ID NO: 3) may be preferred.

Sequences KAEWKSLGEEAWHSK (SEQ ID NO: 1), SIDRVEPYSSTAQVQFD (SEQ ID NO: 2) and SIDRVNPYSSTAQVQFD (SEQ ID NO: 3), all, are capable of direct binding to FGFR and stimulating the receptor, and they all are capable of stimulating learning and memory, However, the sequence KAEWKSLGEEAWHSK (SEQ ID NO: 1) is capable some additional biological activities in comparison to the sequences SIDRVEPYSSTAQVQFD (SEQ ID NO: 2) and SIDRVNPYSSTAQVQFD (SEQ ID NO: 3), namely it is capable of stimulating neurite outgrowth and neural cell survival, which the sequences SIDRVEPYSSTAQVQFD (SEQ ID NO: 2) and sequence SIDRVNPYSSTAQVQFD (SEQ ID NO: 3) are not capable. The inventors identify herein a structural feature which confers to a sequence the capability of both binding and activating FGFR, stimulating learning and memory, stimulating neurite outgrowth and neural cell survival. This feature is that a peptide sequence which is capable of binding and activating FGFR, stimulating learning and memory, stimulating neurite outgrowth and neural cell survival comprises the amino acid motif of the formula (II): K/R-x^{p}-D/E/N/Q-x^{p}-x₀-S-x₁-x₂-x₃-D/E/N/Q-x^{p}, wherein P (proline residue) at position x^{p} is excluded from the selection of hydrophobic amino acid residues. Thus, the motif of formula (II) defined as

K/R-x^{p}-D/E/N/Q-x^{p}-x₀-S-x₁-x₂-x₃-D/E/N/Q-x^{p},

wherein
x^{p} is a hydrophobic amino acid residue independently selected from A, I, T, V, F or W.,
x₀ is K, R or Y, and
x₁, x₂, x₃ are indepently any amino acid residue,
is a structural motif which confers to a peptide sequence comprising said motif the capability of binding and activating FGFR, stimulating learning and memory, stimulating neurite outgrowth and neural cell survival. In some embodiments, such sequence may have the residue Y at position x₀. However, the residues K or R are preferred at this position. Different embodiments for the residues x₁, x₂, x₃ are discussed above. Preferred embodiments for the residues of the positions x₁, x₂ and x₃ are residues G, V, L, or E, wherein L or V are more preferred at position x₁, G at position x₂ and E at position x₃.

The presence of residue P at position x^{p} according to invention may restrict capabilities of a peptide sequence comprising the motif as above to the capabilities of binding and activating FGFR and stimulating learning and memory, such as for example the capabilities of the sequences SIDRVEPYSSTAQVQFD (SEQ ID NO: 2) and SIDRVNPYSSTAQVQFD (SEQ ID NO: 3).

The present invention relates to fragments or variants of the peptide sequence KAEWKSLGEEAWHSK (SEQ ID No1),
which is in detail described above.
In the present context the invention defines
i) a fragment as a sequence which has at least 40%, more preferably at least 50%, more preferably at least 60%, more preferably at least 70%, more preferably at least 80%, more preferably at least 90%, more preferably at least 95% of the length of a sequence comprising the motif of the sequence of SEQ ID NO: 1;
ii) a variant as an amino acid sequence having at least 60 %, more preferably at least 70%, more preferably at least 80%, more preferably at least 90%, more preferably 95% homology to a sequence comprising the motif of above, in particular to the sequence of SEQ ID NO: 1, or as an amino acid sequence having at least 60 %, more preferably at least 70%, more preferably at least 80%, more preferably at least 90%, more preferably 95% positive amino acid matches compared to a sequence comprising the motif of the sequence of SEQ ID NO: 1. A positive amino acid match is defined herein as the identity or similarity of physical and/or chemical properties of the amino acid residues having the same position in two compared sequences. Preferred positive amino acid matches of the present invention are K to R, E to D, L to M, Q to E, I to V, I to L, A to S, Y to W, K to Q, S to T, N to S and Q to R. Homology of one amino acid sequence with another amino acid is defined as the percentage of identical amino acids in the two collated sequences. The homology of the sequences may be calculated using well known algorithms such as BLOSUM 30, BLOSUM 40, BLOSUM 45, BLOSUM 50, BLOSUM 55, BLOSUM 60, BLOSUM 62, BLOSUM 65, BLOSUM 70, BLOSUM 75, BLOSUM 80, BLOSUM 85, or BLOSUM 90;

It is presumed that a fragment or variant as defined above is capable of binding and activating a receptor of the fibroblast growth factor receptor (FGFR) family comprising fibroblast growth factor receptor-1 (FGFR-1), fibroblast growth factor receptor-2 (FGFR-2), fibroblast growth factor receptor-3 (FGFR-3), fibroblast growth factor receptor-4 (FGFR-4) and fibroblast growth factor receptor-5 (FGFR-5), in particular fibroblast growth factor receptor-1 (FGFR-1). Alternatively, it is presumed that a fragment or variant as defined above is capable of stimulating neuronal differentiation, neuronal survival and/or neuronal plasticity.

According to the present invention an isolated peptide sequence as described above may be formulated as a compound.

A compound may contain a single copy of an individual amino acid sequence selected from any of the described above, or it may contain two or more copies of such amino acid sequences. This means that compound of the invention may be formulated as a monomer of a peptide sequence (monomeric compound), such as containing a single individual peptide sequence, or it may be formulated as a multimer of a peptide sequence (multimeric compound), i.e containing two or more individual peptide sequences, wherein said individual peptide sequences may be represented by two or more copies of the same sequence or by two or more different individual peptide sequences. A multimer may also comprise a combination of the full-length sequence and one or more fragments thereof. In one embodiment a compound may contain two amino acid sequences, such compound is defined herein as dimer, in another embodiment a compound may contain more then two amino acid sequences, such for example three, four or more sequences. The present invention preferably relates to compounds containing two or four peptide sequences. However, compounds containing 3, 5, 6, 7, 8 or more sequences are also in the scope of the invention.

As mentioned above, a multimeric compound may comprise the identical peptide sequences, or the sequences may be different. One example of a compound wherein the sequences are different may be a compound containing SEQ ID NO: 1 and SEQ ID NO: 2, another example may be a compound containing SEQ ID NO: 1 and SEQ ID NO: 3. Any combination of the sequences of the invention may be made depending on different embodiments of the invention. The sequences may be connected to each other via a bond, for example the peptide bond, or connected to each other through a linker molecule or grouping. Compounds wherein the peptide sequences are connected through a linker molecule or grouping are preferred. In another embodiment, compound may contain two or more identical copies of a sequence, such as for example two copies of a sequence selected from SEQ ID NO: 1, 2 or 3, wherein said two sequences are connected to each other via a linker molecule or grouping. It is preferred a compound, wherein the sequences are connected via a linker grouping. One example of such linking grouping may be an achiral di-, tri- or tetracarboxylic acid. Suitable achiral di-, tri- or tetracarboxylic acids and a method of producing such a compound, a ligand presentation assembly method (LPA), are discussed in detail further in the specification of the invention. Another example of a possible linker may be the amino acid lysine. Individual peptide sequences may be attached to a core molecule such as lysine forming thereby a dendritic multimer (dendrimer) of an individual peptide sequence(s). Production of dendrimers is well known in the art (PCT/US90/02039, Lu et al., (1991) Mol Immunol. 28:623-630; Defoort et al., (1992) Int J Pept Prot Res. 40:214-221; Drijfhout et al. (1991) Int J Pept Prot Res. 37:27-32), and dedrimers are at present widely used in research and in medical applications. It is a preferred embodiment of the invention to provide a dendrimeric compound comprising four individual amino acid sequences attached to the lysine core molecule. It is also preferred that at least one of the four individual amino acid sequences comprises an amino acid sequence of the formula defined above. It is even more preferred if each of four individual amino acid sequences of a dendrimeric compound individually comprises an amino acid motif selected from the motifs discussed above.

Multimeric compounds of the invention, such as LPA-dimers or Lysin-dendrimers, are preferred compounds of the invention. However, other types of multimeric compounds comprising two or more individual sequences of the invention may be preferred depending on the embodiments.

Multimeric compounds, which comprise one or more copies of an individual sequence of the invention, or fragment, variant or homologue thereof, and another biologically active entity, for example another peptide sequence, are also in the scope of the invention. Among such compounds most preferable are those which comprise at least one peptide sequence of the invention and a peptide sequence which has a capability of stimulating fibroblast growth factor receptor, for example such as peptide sequence EVYVVAENQQGKSKA (SEQ ID NO: 4) or peptide sequence TIMGLKPETRYAVR (SEQ ID NO: 5).

Thus, in one preferred embodiment the multimeric compound of the invention is a dimer comprising two identical copies of an individual peptide sequence comprising a motif of the invention. In another preferred embodiment the multimeric compound of the invention is a dimer comprising two identical copies of an individual peptide sequence comprising a motif of the invention. Still, in another preferred embodiment the multimeric compound is a dimer comprising two different peptide sequences comprising a motif of the invention. A preferred multimeric compound may also be a compound comprising a sequence comprising a motif of he invention and a sequence selected from. TIMGLKPETRYAVR (SEQ ID NO: 5) or EVYVVAENQQGKSKA (SEQ ID NO: 4). Yet, the multimeric compound which is a dendrimer comprising four identical copies of a peptide sequence comprising a motif of the invention linked to a backbone structure of three lysine residues is also among preferred multimeric compounds of the invention.

Orientation of peptide sequences in a multimeric compound may be either N to C, C to N or mixed. By the term "N to C orientation" is meant that two predetermined sequences are connected to a linker through their N-terminal amino acid residue, such as a compound of the type:(COOH)peptide sequence(NH2)-linker-(NH2)peptide sequence(COOH). The term "C to N orientation" is meant that two predetermined sequences are connected to a linker through their C-terminal amino acid residue, such as a compound of the type:(NH2)peptide sequence(COOH)-linker-(COOH)peptide sequence(NH2). The term "mixed orientation" is meant that two predetermined sequences are connected to a linker either through their C-terminal amino acid residue or N-terminal, such as one peptide sequence though its N-terminal amino acid residue and another through its C-terminal amino acid residue, for example as a compound of the type:(NH2)peptide sequence(COOH)-linker-(NH2)peptide sequence(COOH).

According to the invention a peptide sequence as defined above and compound comprising said sequence are capable of binding and activation FGFR.

### 2. Biological activity

Thus, peptide sequences of the invention are capable of binding and activating a functional cell-surface receptor, which is a receptor of the fibroblast growth factor receptor (FGFR) family comprising fibroblast growth factor receptor-1 (FGFR-1), fibroblast growth factor receptor-2 (FGFR-2), fibroblast growth factor receptor-3 (FGFR-3), fibroblast growth factor receptor-4 (FGFR-4) and fibroblast growth factor receptor-5 (FGFR-5), preferably, FGFR-1.

Extracellular binding of a ligand to a receptor leads to receptor activation which means that the receptor is becoming capable of activating at least one intracellular signal transduction pathway resulting in one or another cellular response.

The term "binding" refers herein to a direct interaction of a peptide sequence of the invention with FGFR. By the term "interaction" is meant that a peptide sequence has a transient or permanent direct contact with FGFR. The wording "activation of the receptor" means that the receptor become capable of transmitting the effect of "ligand binding" into a cascade of biochemical reactions collectively termed "receptor signalling" or "signal transduction" inside the cell resulting in a physiological response of the cell.

Peptide sequences of the invention which are capable of binding and activating a cellular receptor are thus capable of mimicking the action of natural receptor ligands and, in this context, they should be understood as mimics of the receptor ligands, in particular ligands of FGFR. It is known that, a receptor may have a number of different ligands, binding of which to the receptor activates different signal transduction pathways and results in different cellular responces.

The invention preferably concerns FGFR-1 associated signal transduction pathways. Activation of FGFR-1 is defined herein by the degree of FGFR-1 tyrosine phosphorylation, which is according to the present invention increases due to the binding of a sequence of the invention. Activation of an FGFR-1 associated intracellular signal transduction pathway is defined herein as the presence of an activated molecule of one or more intracellular proteins involved in a FGFR-1 associated signal transduction pathway, such as for example activated molecules of proteins STAT1, JNK, PLCγ, ERK, STAT5, PI3K, PKC, FRS2 and/or GRB2. The activated status of a molecule of one or more intracellular proteins involved in a FGFR-1 associated signal transduction pathway is defined herein as the degree of phosphorylation of said molecule, which is according to the present invention increases or desreases due to the binding of a sequence of the invention to FGFR. The degree of phosphorylation is estimated as at least 20% above/below the control value, such as at least 20-200 %, for example at least 50-200%. The control value is estimated as a degree of phosphorylation of the protein of interest when a compound capable of binding and activating FGFR is absent in cell environment. When concerned the FGFR-1 signalling dependent cellular response, the invention in particular relates to a cellular response selected from but not limited by
i) induction of cell differentiation, such as for example differentiation of neuronal cells, stem cells or cancer cells, nerve regeneration, neurite outgrowth and branching, inhibition of cell proliferation;
ii) increased cell survival, such for example neuronal cell survival due body traumatic damage or due to disease, inhibition of apoptosis;
iii) increased cellular plasticity, for example morphological plasticity, such as for example neural cell plasticity associated with the memory and learning.

A peptide sequence of the invention may derive from the neural cell adhesion molecule (NCAM), for example from NCAM polypeptide identified in the GenBank database under Swiss-Prot Ass Nos.: P13591, P13592, P13596 or P13595. An example of such peptide sequence may be sequence KAEWKSLGEEAWHSK (SEQ ID NO: 1). These peptide sequences are the integral sequences of NCAM polypeptide and when taken as isolated peptide sequences they are capable according to the invention of mimicking activity of NCAM associated with FGFR function, such as stimulating NCAM-FGFR-dependent neural cell differentiation, survival and plasticity. However, the invention does not limit capability of a peptide sequence of the invention to mimicking the function of NCAM. Recently, it has been described a new group of low-affinity ligands of FGFR that are capable of binding to the receptor with the affinity similar to NCAM binding and to the same binding site on the receptor molecule as NCAM (WO 04/056865). According to the present invention, the peptide sequences of NCAM described herein and the peptide sequences of NCAM described in WO 04/056865 represent different parts of NCAM binding site for FGFR. Via this binding site NCAM interacts with a binding site on FGFR which is common for the low-affinity FGFR ligands described in WO 04/056865. Therefore, a peptide sequence of the present invention may be capable of mimicking the binding of a low-affinity ligand from the latter group of FGFR ligands to FGFR and thus mimicking biological activity of said ligand. Thus, the invention includes in the scope any biological activity of the following proteins associated with FGFR function:
Neural cell adhesion molecule L1 (of Swiss-Prot Ass. Nos: Q9QYQ7, Q9QY38, P11627, Q05695, or P32004),
   - Neural Cell Adhesion Molecule-2 (NCAM-2) (of Swiss-Prot Ass. No: P36335)
   - Neuron-glia Cell Adhesion Molecule (Ng-CAM) (of Swiss-Prot Ass. No: Q03696 or Q90933),
   - Neural cell adhesion molecule CALL (of Swiss-Prot Ass. No: 000533),
   - Neuroglian (of Swiss-Prot Ass. No: P91767or P20241),
   - Nr-CAM (HBRAVO, NRCAM, NR-CAM 12) (of Swiss-Prot Ass. Nos: Q92823, O15179 or Q9QVN3),
   - Axonin-1/TAG-1 (of Swiss-Prot Ass. Nos: Q02246, P22063 or P28685),
   - Axonal-associated Cell Adhesion Molecule (AxCAM) (of Swiss-Prot Ass. No: Q8TC35 or NP_031544.1),
   - Myelin-Associated Glycoprotein (MAG) (of Swiss-Prot Ass. No: P20917),
   - Neural cell adhesion molecule BIG-1 (of Swiss-Prot Ass. No: Q62682),
   - Neural cell adhesion molecule BIG-2 (of Swiss-Prot Ass. No: Q62845),
   - Fasciclin (FAS-2) (of Swiss-Prot Ass. No: P22648),
   - Neural cell adhesion molecule HNB-3/NB-3 (of Swiss-Prot Ass. Nos: Q9UQ52, P97528 or Q9JMB8)
   - Neural cell adhesion molecule HNB-2/NB-2 (of Swiss-Prot Ass. Nos: 094779, P07409 or P97527),
   - Cadherin (of Swiss-Prot Ass. No: Q9VW71),
   - Junctional Adhesion Molecule-1 (JAM-1) (of Swiss-Prot Ass. Nos: Q9JKD5 or 088792),
   - Neural cell adhesion F3/F11 (Contactin) (of Swiss-Prot Ass. Nos: Q63198, P1260, Q12860, Q28106, P14781 or 093250),
   - Neurofascin (of Swiss-Prot Ass. Nos: Q90924, Q91Z60 or 042414),
   - B-lymphocyte cell adhesion molecule CD22 (of Swiss-Prot Ass. Nos: Q9R094 or P20273),
   - Neogenin (NEO1) (of Swiss-Prot Ass. Nos: Q92859, P97603, Q90610 or P97798),
   - Intercellular Cell Adhesion Molecule-5 (ICAM-5/telencephalin) (of Swiss-Prot Ass. No: Q8TAM9, Q60625),
   - Galactose binding lectin-12 (galectin-12) (of Swiss-Prot Ass. Nos: Q91 VD1, Q9JKX2 or Q9NZ03),
   - Galactose binding lectin-4 (galectin-4) (of Swiss-Prot Ass. No: Q8K419; P38552),
   - Fibroblast Growth Factor Receptor 1 (FGFR1) (of Swiss-Prot Ass. Nos: Q9QZM7, Q99AW7, Q9UD50 or Q63827),
   - Fibroblast Growth Factor Receptor 2 (FGFR2) (of Swiss-Prot Ass. Nos: Q96KM2, P21802 or Q63241),
   - Fibroblast Growth Factor Receptor 3 (FGFR3) (of Swiss-Prot Ass. Nos: Q95M13, AF487554 or Q99052),
   - Fibroblast Growth Factor Receptor 4 (FGFR4) (of Swiss-Prot Ass. No: Q91742),
   - Neurotrophin Tyrosin Kinase Type-2 (NTRKT-2) (Swiss-Prot Ass. No: Q8WXJ5),
   - Leukocyte Antigen Related Protein-Tyrosine Phosphatase (LAR-PTPRF) (of Swiss-Prot Ass. Nos: Q9EQ17, Q64605, Q64604, Q9QW67, Q9VIS8 or P10586),
   - Nephrin (of Swiss-Prot Ass. Nos: Q925S5, Q9JIX2, Q9ET59, Q9R044, or Q9QZS7, Q06500),
   - Protein-Tyrosine Phosphatase Receptor type S (PTPRS) (of Swiss-Prot Ass. Nos: Q64699, Q13332 or 075870),
   - Protein-Tyrosine Phosphatase Receptor type kappa (R-PTP-kappa) (of Swiss-Prot Ass. No: Q15262),
   - Protein-Tyrosine Phosphatase Receptor type D (PTPRD) (of Swiss-Prot Ass. Nos: Q8WX65, Q91AJ1, P23468 or Q64487),
   - Ephrin type-A receptor 8 (EPHA8/Tyrosine-Protein Kinase Receptor EEK) (of Swiss-Prot Ass. Nos: 009127 or P29322),
   - Ephrin type-A receptor 3 (EPHA8/Tyrosine-Protein Kinase Receptor ETK-1/CEK4) (of Swiss-Prot Ass. No: P29318),
   - Ephrin type-A receptor 2 (of Swiss-Prot Ass. No: Q8N3Z2)
   - Insulin Receptor (IR) (of Swiss-Prot Ass. No: Q9PWN6)
   - Insulin-like Growth Factor-1 Receptor (IGF-1) (of Swiss-Prot Ass. Nos: Q9QVW4, P08069, P24062, Q60751, P15127 or P15208)
   - Insulin-related Receptor (IRR) (of Swiss-Prot Ass. No: P14616),
   - Tyrosine-Protein Kinase Receptor Tie-1 (of Swiss-Prot Ass. Nos: 06805, P35590 or Q06806),
   - Roundabout receptor-1 (robo-1) (of Swiss-Prot Ass. Nos: 044924, AF041082 or Q9Y6N7),
   - Neuronal nicotinic acetylcholine receptor alpha 3 subunit (CHRNA3) (of Swiss-Prot Ass. Nos: Q8VHH6, P04757, Q8R4G9 or P32297)
   - Neuronal acetylcholine receptor alpha 6 subunit (of Swiss-Prot Ass. Nos: Q15825 or Q9R0W9)
   - Platelet-Derived Growth Factor Receptor Beta (PDGFRB) (of Swiss-Prot Ass. Nos: Q8R406 or Q05030),
   - Interleukin-6 Receptor (IL-6R) (of Swiss-Prot Ass. No: Q00560),
   - Interleukin-23 Receptor (IL-23R) (of Swiss-Prot Ass. No: AF461422),
   - Beta-common cytokine receptor of IL-3, IL5 and GmCsf (of Swiss-Prot Ass. No: P32927)
   - Cytokine Receptor-Like molecule 3 (CRLF1) (of Swiss-Prot Ass. No: Q9JM58),
   - Class I Cytokine Receptor (ZCYTOR5) (of Swiss-Prot Ass. No: Q9UHH5)
   - Netrin-1 receptor DCC (of Swiss-Prot Ass. No: P43146),
   - Leukocyte Fc Receptor-like Protein (IFGP2) (of Swiss-Prot Ass. Nos: Q96PJ6 or Q96KM2),
   - Macrophage Scavenger Receptor 2 (MSR2) (of Swiss-Prot Ass. No: Q91YK7),
   - Granulocyte Colony Stimulating Factor Receptor (G-CSF-R) (of Swiss-Prot Ass. No: Q99062),
   - perlecan (of Swiss-Prot Ass. No: P98160),
   - ADAM-8 (of Swiss-Prot Ass. No: Q05910),
   - ADAM-19 (of Swiss-Prot Ass. Nos: Q9H013 or 035674),
   - ADAM-8 (of Swiss-Prot Ass. No: P78325),
   - ADAM-12 (of Swiss-Prot Ass. Nos: 043184 or Q61824),
   - ADAM-28 (of Swiss-Prot Ass. Nos: Q9JLN6, Q61824, Q9XSL6 or Q9UKQ2),
   - ADAM-33 precursor (of Swiss-Prot Ass. Nos: Q8R533 or Q923W9),
   - ADAM-9 (of Swiss-Prot Ass. Nos: Q13433 or Q61072),
   - ADAM-7 (of Swiss-Prot Ass. NoS: Q9H2U9, 035227 or Q63180),
   - ADAM-1 A Fertilin alpha (of Swiss-Prot Ass. No: Q8R533),
   - ADAM-15 (of Swiss-Prot Ass. Nos: Q9QYV0, 088839 or Q13444),
   - Metalloproteinase-desintegrin domain containing protein (TECAM) (o fSwiss-Prot Ass. No: AF163291),
   - Metalloproteinase 1 (of Swiss-Prot Ass. Nos: 095204 or Q9BSI6),
   - Collagen type VII (Swiss-Prot Ass. No: Q63870),
   - Fibronectin (of Swiss-Prot Ass. Nos: Q95KV4, Q95KV5, P07589, Q28377, U42594, 095609 or P11276),
   - Tenascin-R (of Swiss-Prot Ass. Nos: Q15568, 000531, Q90995 of P10039),
   - Cytokine-like factor-1 (CLF-1) (of Swiss-Prot Ass. No:075462)

Any interaction between two molecules may be characterised by the affinity of interaction, which means the strength of attraction between two molecules. The affinity of interaction is commonly expressed by the value of Kd, a dissociation equilibrium constant. Kd reflects the ratio between the rate of dissociation and the rate of binding between two molecules and thus represents a measure of the strength of binding between two molecules. The stronger binding is reflected by the lower value of Kd.

The above FGFR ligands have a relatively low affinity binding to FGFR. The low affinity interaction of the invention is characterised by Kd having a value in the range of 10⁻³ to 10⁻¹⁰ M, such for example in the range of 10⁻⁴ to 10⁻⁸ M. Thus, the invention relates to affinity of interaction between a peptide sequence and FGFR in the range of 10⁻³ to 10⁻¹⁰ M, preferably in the range of 10⁻⁴ to 10⁻⁸ M.

In different embodiments the invention may relate to different biological activities of the peptide sequences of the invention. Thus in one embodiment, the invention may relate to the capability of a peptide sequence to stimulate neural plasticity associated with learning and memory. According to the invention, such peptide sequence may be selected from any peptide sequences described herein. A preferred sequence may sequence KAEWKSLGEEAWHSK (SEQ ID NO: 1), or sequence SIDRVEPYSSTAQVQFD (SEQ ID NO: 2).

In another embodiment the invention may relate to a capability of a peptide sequence to stimulate neuronal differentiation such as differentiation of neural precursor cells or differentiation of neurons which includes neurite outgrowth and/or branching of the processes. According to the invention this activity is associated with a peptide sequence which comprises the motif K/R-x^{p}-D/E/N/Q-x^{p}-x₀-S-x₁-x₂-x₃-D/E/N/Q-x^{p}, as defined in claim 37.

Accordingly, a preferred sequence of such embodiment may be the sequence KAEWKSLGEEAWHSK (SEQ ID NO: 1). A peptide sequence comprising the above motif is also capable of stimulating cell survival, preferably neuronal cell survival and therefore some preferred embodiments of the invention may relate to a capability of a peptide sequence to stimulate cell survival, preferably neuronal cell survival. A preferred sequence of such embodiments may also be the sequence KAEWKSLGEEAWHSK (SEQ ID NO: 1). A sequence comprising the above motif may also be preferred when the invention relates to a sequence which is capable two or more biological activities selected from
i) stimulating learning and memory,
ii) stimulating neural cell survival,
iii) stimulating neural cell differentiation.

A preferred sequence of such embodiments is also the sequence KAEWKSLGEEAWHSK (SEQ ID NO: 1).

### 3. Production of peptide fragments

The peptides of the present invention may be prepared by any conventional synthetic methods, recombinant DNA technologies, enzymatic cleavage of full-length proteins which the peptide sequences are derived from, or a combination of said methods.

### Recombinant preparation

Thus, in one embodiment the peptides of the invention are produced by use of recombinant DNA technologies.

The DNA sequence encoding a peptide or the corresponding full-length protein the peptide originates from may be prepared synthetically by established standard methods, e.g. the phosphoamidine method described by Beaucage and Caruthers, 1981, Tetrahedron Lett. 22:1859-1869, or the method described by Matthes et al., 1984, EMBO J. 3:801-805. According to the phosphoamidine method, oligonucleotides are synthesised, e.g. in an automatic DNA synthesiser, purified, annealed, ligated and cloned in suitable vectors.

The DNA sequence encoding a peptide may also be prepared by fragmentation of the DNA sequences encoding the corresponding full-length protein of peptide origin, using DNAase I according to a standard protocol (Sambrook et al., Molecular cloning: A Laboratory manual. 2 rd ed., CSHL Press, Cold Spring Harbor, NY, 1989). The DNA encoding the full-length protein may alternatively be fragmented using specific restriction endonucleases. The fragments of DNA are further purified using standard procedures described in Sambrook et al., Molecular cloning: A Laboratory manual. 2 rd ed., CSHL Press, Cold Spring Harbor, NY, 1989.

The DNA sequence encoding a full-length protein may also be of genomic or cDNA origin, for instance obtained by preparing a genomic or cDNA library and screening for DNA sequences coding for all or part of the full-length protein by hybridisation using synthetic oligonucleotide probes in accordance with standard techniques (cf. Sambrook et al., Molecular Cloning: A Laboratory Manual, 2nd Ed., Cold Spring Harbor, 1989). The DNA sequence may also be prepared by polymerase chain reaction using specific primers, for instance as described in US 4,683,202 or Saiki et al., 1988, Science 239:487-491.

The DNA sequence is then inserted into a recombinant expression vector, which may be any vector, which may conveniently be subjected to recombinant DNA procedures. The choice of vector will often depend on the host cell into which it is to be introduced. Thus, the vector may be an autonomously replicating vector, i.e. a vector that exists as an extrachromosomal entity, the replication of which is independent of chromosomal replication, e.g. a plasmid. Alternatively, the vector may be one which, when introduced into a host cell, is integrated into the host cell genome and replicated together with the chromosome(s) into which it has been integrated.

In the vector, the DNA sequence encoding a peptide or a full-length protein should be operably connected to a suitable promoter sequence. The promoter may be any DNA sequence, which shows transcriptional activity in the host cell of choice and may be derived from genes encoding proteins either homologous or heterologous to the host cell. Examples of suitable promoters for directing the transcription of the coding DNA sequence in mammalian cells are the SV 40 promoter (Subramani et al., 1981, Mol. Cell Biol. 1:854-864), the MT-1 (metallothionein gene) promoter (Palmiter et al., 1983, Science 222: 809-814) or the adenovirus 2 major late promoter. A suitable promoter for use in insect cells is the polyhedrin promoter (Vasuvedan et al., 1992, FEBS Lett. 311:7-11). Suitable promoters for use in yeast host cells include promoters from yeast glycolytic genes (Hitzeman et al., 1980, J. Biol. Chem. 255:12073-12080; Alber and Kawasaki, 1982, J. Mol. Appl. Gen. 1: 419-434) or alcohol dehydrogenase genes (Young et al., 1982, in Genetic Engineering of Microorganisms for Chemicals, Hollaender et al, eds., Plenum Press, New York), or the TPI1 (US 4,599,311) or ADH2-4c (Russell et al., 1983, Nature 304:652-654) promoters. Suitable promoters for use in filamentous fungus host cells are, for instance, the ADH3 promoter (McKnight et al., 1985, EMBO J. 4:2093-2099) or the tpiA promoter.

The coding DNA sequence may also be operably connected to a suitable terminator, such as the human growth hormone terminator (Palmiter et al., op. cit.) or (for fungal hosts) the TPI1 (Alber and Kawasaki, op. cit.) or ADH3 (McKnight et al., op. cit.) promoters. The vector may further comprise elements such as polyadenylation signals (e.g. from SV 40 or the adenovirus 5 Elb region), transcriptional enhancer sequences (e.g. the SV 40 enhance) and translational enhancer sequences (e.g. the ones encoding adenovirus VA RNAs).

The recombinant expression vector may further comprise a DNA sequence enabling the vector to replicate in the host cell in question. An example of such a sequence (when the host cell is a mammalian cell) is the SV 40 origin of replication. The vector may also comprise a selectable marker, e.g. a gene the product of which complements a defect in the host cell, such as the gene coding for dihydrofolate reductase (DHFR) or one which confers resistance to a drug, e.g. neomycin, hydromycin or methotrexate.

The procedures used to ligate the DNA sequences coding the peptides or full-length proteins, the promoter and the terminator, respectively, and to insert them into suitable vectors containing the information necessary for replication, are well known to persons skilled in the art (cf., for instance, Sambrook et al., op.cit.).

To obtain recombinant peptides of the invention the coding DNA sequences may be usefully fused with a second peptide coding sequence and a protease cleavage site coding sequence, giving a DNA construct encoding the fusion protein, wherein the protease cleavage site coding sequence positioned between the HBP fragment and second peptide coding DNA, inserted into a recombinant expression vector, and expressed in recombinant host cells. In one embodiment, said second peptide selected from, but not limited by the group comprising glutathion-S-reductase, calf thymosin, bacterial thioredoxin or human ubiquitin natural or synthetic variants, or peptides thereof. In another embodiment, a peptide sequence comprising a protease cleavage site may be the Factor Xa, with the amino acid sequence *IEGR,* enterokinase, with the amino acid sequence *DDDDK,* thrombin, with the amino acid sequence *LVPR*/*GS,* or *Acharombacter lyticus,* with the amino acid sequence *XKX,* cleavage site.

The host cell into which the expression vector is introduced may be any cell which is capable of expression of the peptides or full-length proteins, and is preferably a eukaryotic cell, such as invertebrate (insect) cells or vertebrate cells, e.g. *Xenopus laevis* oocytes or mammalian cells, in particular insect and mammalian cells. Examples of suitable mammalian cell lines are the HEK293 (ATCC CRL-1573), COS (ATCC CRL-1650), BHK (ATCC CRL-1632, ATCC CCL-10) or CHO (ATCC CCL-61) cell lines. Methods of transfecting mammalian cells and expressing DNA sequences introduced in the cells are described in e.g. Kaufman and Sharp, J. Mol. Biol. 159, 1982, pp. 601-621; Southern and Berg, 1982, J. Mol. Appl. Genet. 1:327-341; Loyter et al., 1982, Proc. Natl. Acad. Sci. USA 79: 422-426; Wigler et al., 1978, Cell 14:725; Corsaro and Pearson, 1981, in Somatic Cell Genetics 7, p. 603; Graham and van der Eb, 1973, Virol. 52:456; and Neumann et al., 1982, EMBO J. 1:841-845.

Alternatively, fungal cells (including yeast cells) may be used as host cells. Examples of suitable yeast cells include cells of *Saccharomyces spp. or Schizosaccharomyces spp.,* in particular strains of *Saccharomyces cerevisiae.* Examples of other fungal cells are cells of filamentous fungi, e.g. *Aspergillus spp.* or *Neurospora spp.,* in particular strains of *Aspergillus oryzae* or *Aspergillus niger.* The use of *Aspergillus spp.* for the expression of proteins is described in, e.g., EP 238 023.

The medium used to culture the cells may be any conventional medium suitable for growing mammalian cells, such as a serum-containing or serum-free medium containing appropriate supplements, or a suitable medium for growing insect, yeast or fungal cells. Suitable media are available from commercial suppliers or may be prepared according to published recipes (e.g. in catalogues of the American Type Culture Collection).

The peptides or full-length proteins recombinantly produced by the cells may then be recovered from the culture medium by conventional procedures including separating the host cells from the medium by centrifugation or filtration, precipitating the proteinaceous components of the supernatant or filtrate by means of a salt, e.g. ammonium sulphate, purification by a variety of chromatographic procedures, e.g. HPLC, ion exchange chromatography, affinity chromatography, or the like.

### Synthetic preparation

The methods for synthetic production of peptides are well known in the art. Detailed descriptions as well as practical advice for producing synthetic peptides may be found in Synthetic Peptides: A User's Guide (Advances in Molecular Biology), Grant G. A. ed., Oxford University Press, 2002, or in: Pharmaceutical Formulation: Development of Peptides and Proteins, Frokjaer and Hovgaard eds., Taylor and Francis, 1999.

Peptides may for example be synthesised by using Fmoc chemistry and with Acm-protected cysteins. After purification by reversed phase HPLC, peptides may be further processed to obtain for example cyclic or C- or N-terminal modified isoforms. The methods for cyclization and terminal modification are well-known in the art and described in detail in the above-cited manuals.

In a preferred embodiment the peptide sequences of the invention are produced synthetically, in particular, by the Sequence Assisted Peptide Synthesis (SAPS) described in the above manuals.

Otherwise, the synthesis of an individual peptide sequence of the invention may be ordered and purchased from a commercial manufacturer, such as for example Sigma-Genosys (USA).

### Production of multimers of individual peptide sequences by the LPA method

The LPA method is disclosed in WO 00/18791. The method essentially comprises the following steps:
(a) providing by solid phase synthesis or fragment coupling peptide fragment(s) comprising the desired sequence(s), the peptide fragment(s) being attached to a solid phase;
(b) if necessary, deprotecting any N-terminal amino groups whole the peptide fragment(s) are still attached to a solid phase,
(c) reacting the peptide fragment(s) having unprotected N-terminal groups with an achiral di-, tri-, or tetracarboxylic acid so as to provide a construct having a ring structure, and
(d) cleaving the construct from the solid phase so as to provide an LPA comprising the peptide fragment(s) having free C-terminal groups.

In the above method, prior step (d) the following steps may be performed:
(c1) if present, deprotecting any N-protected groups originating from the carboxylic acid used in step (c),
(c2) continuing the solid phase synthesis or fragment coupling so as to provide peptide fragment(s) comprising desired sequence(s) having at least one N-protected N-terminal amino acid group and/or attaching chemical moieties, and
(c3) deprotecting, if present, any N-terminal amino groups (prior or after step (d)).

The method provides i.a. LPAs presenting desired sequences of the invention with N to C orientation (step (c)). And also simultaneously sequences with C to n orientation (step (c2))

Thus, to obtain a compound of the invention two peptide chains attached to a solid phase are to be assembled by means of achiral di-, tri- or tetracarboxylic acids. Suitable achiral di-, tri- or tetracarboxylic acids to be used in the present method have the general formula

X[(A)nCOOH][(B)mCOOH]

wherein n and m independently are an integer of from 1 to 20, X is HN, A and B independently are a substituted or unsubstituted C₁₋₁₀ alkyl, a substituted or unsubstituted C₂₋₁₀ alkenyl, a substituted or unsubstituted cyclic moiety, a substituted or unsubstituted heterocyclic moiety, a substituted or unsubstituted aromatic moiety, or A and B together form a substituted or unsubstituted cyclic moiety, substituted or unsubstituted heterocyclic moiety, substituted or unsubstituted aromatic moiety.

In another embodiment suitable achiral di-, tri- or tetracarboxylic acids to be used in the present method have the general formula

X[(A)nCOOH][(B)mCOOH]

wherein n and m are 0 or an integer of from 1 to 20, X is H₂N(CR₂)pCR, or RHN(CR₂)pCR, wherein p is 0 or integer of from 1 to 20, wherein each R is H, a substituted or unsubstituted C₁₋₁₀ alkyl, a substituted or unsubstituted C₂₋₁₀ alkenyl, a substituted or unsubstituted cyclic moiety, a substituted or unsubstituted heterocyclic moiety, a substituted or unsubstituted aromatic moiety, or A and B together form a substituted or unsubstituted cyclic moiety, substituted or unsubstituted heterocyclic moiety, substituted or unsubstituted aromatic moiety.

In still another embodiment suitable achiral di-, tri- or tetracarboxylic acids to be used in the present method have the general formula

X[(A)nCOOH][(B)mCOOH]

wherein n and m are 0 or an integer of from 1 to 20, X is HO(CR₂)pCR, HS(CR₂)pCR, halogen-(CR₂)pCR, HOOC(CR₂)pCR, ROOC(CR₂)pCR, HCO(CR₂)pCR, RCO(CR₂)pCR, or [HOOC(A)n][HOOC(B)m]CR(CR₂)pCR, wherein p is 0 or integer of from 1 to 20, each R independently is H or a substituted or unsubstituted C₁₋₁₀ alkyl, a substituted or unsubstituted C₂₋₁₀ alkenyl, a substituted or unsubstituted cyclic moiety, a substituted or unsubstituted heterocyclic moiety, a substituted or unsubstituted aromatic moiety, or A and B together form a substituted or unsubstituted cyclic moiety, substituted or unsubstituted heterocyclic moiety, substituted or unsubstituted aromatic moiety.
In yet another embodiment suitable achiral di-, tri- or tetracarboxylic acids to be used in the present method have the general formula

X[(A)nCOOH][(B)mCOOH]

Wherein n and m are 0 or an integer of from 1 to 20, X is H₂N(CR₂)p, RHN(CR₂)p, HO(CR₂)p, HS(CR₂)p, halogen-(CR₂)p, HOOC(CR₂)p, ROOC(CR₂)p, HCO(CR₂)p, RCO(CR₂)p, or [HOOC(A)n][HOOC(B)m](CR₂)p, wherein p is 0 or integer of from 1 to 20, each R independently is H or a substituted or unsubstituted C₁₋₁₀ alkyl, a substituted or unsubstituted C₂₋₁₀ alkenyl, a substituted or unsubstituted cyclic moiety, a substituted or unsubstituted heterocyclic moiety, a substituted or unsubstituted aromatic moiety, or A and B together form a substituted or unsubstituted cyclic moiety, substituted or unsubstituted heterocyclic moiety, substituted or unsubstituted aromatic moiety.

Under the term C₁₋₁₀ alkyl is meant straight or branched chain alkyl groups having 1-10 carbon atoms, e.g. methyl, ethyl, isopropyl, butyl, and tertbutyl.

Under the term C₂₋₁₀ alkenyl is meant straight or branched chain alkenyl groups having 2-10 carbon atoms, e.g. ethynyl, propenyl, isopropenyl, butenyl, and tert-butenyl.

Under the term cyclic moiety is meant cyclohexan, and cyclopentane.

Under the term aromatic moiety is meant phenyl.

The wording "A and B forms a cyclic, heterocyclic or aromatic moiety" denotes cyclohexan, piperidine, benzene, and pyridine.

By reaction with a carboxylic acid, a construct of the type
X(CO-sequence)2-solid phase, wherein X as defined above,
is obtained.

By the term "peptide" is in the present content meant a peptide comprising naturally occurring and/or non-naturally occurring amino acids, a PNA-sequence, or peptidomimetic. By "naturally occurring amino acids" is meant L- and D-forms of the 20 acids found in nature. Non-naturally occurring amino acids are e.g. modified naturally occurring amino acids. The term sequence is further intended to comprise one or more of such sequences. Examples of suitable peptidomimetics are described in Marshall G.R.,(1993) Tetrahedron, 49:3547-3558. The term "chemical moieties" denotes an entity enhancing the solubility or biological activity of the LPA, and entity for directing the LPA to its target or a marker. Preferred embodiments for the sequences are described above.

The group X permits directly or indirectly continued stepwise synthesis or a fragment coupling of the same sequence, or of one or more different sequences and/or moieties. Orientation of peptide fragments (N to C or C to N) in LPA is defined as desired. In one embodiment the present invention features LPAs with N to C orientation, in another embodiment it concerns the compounds with simultaneous N to C and C to N presentation of the sequences, and in yet another embodiment the sequences have C to N orientation.

In the case where X comprises a temporally protected amino function, synthesis or coupling can be carried out directly after protection. Suitable activation of all carboxyl-containing groups providing effective formation of the ring system (on step (c), see above) can be ensured using half-equivalent carboxy acid. In case of tri- or tetracarboxylic acids the activated carboxy group may further be derivatised with a diamine such as ethylenediamine or an amine suitably functionalised for further reactions such as mercapto-, an oxy-, an oxo or carboxyl group. In the case of diamine, peptide synthesis or fragment coupling can be continued directly according to the desired sequence or chemical moiety. In a preferred embodiment, the Fmoc-protection strategy is used, but any amino protection group may be used depending on the synthesis or coupling strategy. Examples are the Boc-protection group strategy.

Since the continued stepwise synthesis or fragment coupling is performed with one or in case of a bifunctional chemical moiety such lysine with two amino acid groups, it has surprisingly been found that a much better result can be obtained as compared to conventional tetrameric lysine dendimers obtained by the MAP synthesis. Furthermore, optimal peptide synthesis procedures or coupling procedures can be used for the single chains attached to the solid phase, and their homogeneity can be verified prior to forming the LPA. Cleavage from the solid phase and simultaneous side-chain deprotection can be performed by standard peptide synthesis procedures (described above). A final product may thus be obtained having optimal and well-defined composition. Purification by standard chromatography methods such as HPLC or gel filtration can easily be performed, if desired or needed.

Favourable di-, tri- and tetracarboxilyc acids for providing the ring structure may be selected from imino diacetic acid, 2-amino malonic acid, 3-amino glutaric acid, 3-methylamino glutaric acid, 3-chloro glutamic acid, 3-methoxy-carbonyl glutaric acid, 3-acetyl glutaruc acid, glutaric acid, tricarballylic acid,3,4-bis-carboxymethyl adipic acid, 4-(2-carboxyethyl)-pimelic acid, (3,5-bis-carboxymethyl-phenyl)-acetic acid, 3,4-bis-carboxymethyl-adipic acid, benzene-1,2,4,5-tetra carboxylic acid, 4-(3-carboxy-allylamino)-but-2-enoic acid, 4,4-imino-dibenzoic acid, 1,4-dihydropyridine-3,5-dicarboxylic acid, 5-amino isophthalic acid, 2-chloro malonic acid, 3-hydroxy glutaric acid, and benzene-1,3,5-tricarboxylic acid.

Fragment coupling (fragment coupling or fragment condensation) may be performed according to standard procedures, e.g. as described in Peptide Synthesis protocols, Methods in Molecular Biology Vol. 35, Chapter 15, 303-316, Nyfeler R, Pennington MW and Dunne BM Eds.,Humana Press, 1994. Accordingly, fragments may be synthesised on a solid phase, cleaved from the solid phase with full preservation of protecting groups, purified and characterised as described above. Suitable fragments may also be obtained by other techniques described above.

It is one of preferred embodiments of the invention to use the above described LPA method for the production of a compound of the invention.

### 4. Antibody

It is an objective of the present invention to provide an antibody, antigen binding fragment or recombinant protein thereof capable of recognizing and selectively binding to an epitope comprising or being comprised by a peptide sequence comprising a motif as any of the motifs described above, or fragment, variant or homologue of said sequence. In one preferred embodiment the antibody is an antibody that recognizes and binds to an epitope comprising the motif K/R-x^{p}-D/E/N/Q-x^{p}-x₀-S-x₁-x₂-x₃-D/E/N/Q-x^{p}, wherein x^{p} is any hydrophobic amino acid residue, x₀ is K, R or Y, and x₁, x₂, x₃ are indepently any amino acid residue.

In another preferred embodiment an antibody is able to recognize and bind to an epitope on NCAM, said epitope comprising the motif K/R-x^{p}-D/E/N/Q-x^{p}-x₀-S-x₁-x₂-x₃-D/EJN/Q-x^{p}, wherein x^{p} is W or P, x₀ is K, R or Y, and x₁, x₂, x₃ are independently any amino acid residue. Even more preferred an antibody which recognize and bind to an epitope comprising or being comprised by sequence KAEWKSLGEEAWHSK (SEQ ID NO: 1) and/or sequence SIDRVEPYSSTAQVQFD (SEQ ID NO: 2) of NCAM.

By the term "epitope" is meant the specific group of atoms (on an antigen molecule) that is recognized by (that antigen's) antibodies (thereby causing an immune response). The term "epitope" is the equivalent to the term "antigenic determinant". The epitope may comprise 3 or more amino acid residues, such as for example 4, 5, 6, 7, 8 amino acid residues, located in close proximity, such as within a contiguous amino acid sequence, or located in distant parts of the amino acid sequence of an antigen, but due to protein folding have been approached to each other.

Antibody molecules belong to a family of plasma proteins called immunoglobulins, whose basic building block, the immunoglobulin fold or domain, is used in various forms in many molecules of the immune system and other biological recognition systems. A typical immunoglobulin has four polypeptide chains, containing an antigen binding region known as a variable region and a non-varying region known as the constant region.

Native antibodies and immunoglobulins are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies between the heavy chains of different immunoglobulin isotypes. Each heavy and light chain also has regularly spaced intrachain disulfide bridges. Each heavy chain has at one end a variable domain (VH) followed by a number of constant domains. Each light chain has a variable domain at one end (VL) and a constant domain at its other end. The constant domain of the light chain is aligned with the first constant domain of the heavy chain, and the light chain variable domain is aligned with the variable domain of the heavy chain. Particular amino acid residues are believed to form an interface between the light and heavy chain variable domains (Novotny J, & Haber E. Proc Natl Acad Sci USA. 82(14):4592-6,1985).

Depending on the amino acid sequences of the constant domain of their heavy chains, immunoglobulins can be assigned to different classes. There are at least five (5) major classes of immunoglobulins: IgA, IgD, IgE, IgG and IgM, and several of these may be further divided into subclasses (isotypes), e.g. IgG-1, IgG-2, IgG-3 and IgG-4; IgA-1 and IgA-2. The heavy chains constant domains that correspond to the different classes of immunoglobulins are called alpha (α), delta (δ), epsilon (ε), gamma (γ) and mu (µ), respectively. The light chains of antibodies can be assigned to one of two clearly distinct types, called kappa (κ) and lambda (λ), based on the amino sequences of their constant domain. The subunit structures and three-dimensional configurations of different classes of immunoglobulins are well known.

The term "variable" in the context of variable domain of antibodies, refers to the fact that certain portions of the variable domains differ extensively in sequence among antibodies. The variable domains are for binding and determine the specificity of each particular antibody for its particular antigen. However, the variability is not evenly distributed through the variable domains of antibodies. It is concentrated in three segments called complementarity determining regions (CDRs) also known as hypervariable regions both in the light chain and the heavy chain variable domains.

The more highly conserved portions of variable domains are called the framework (FR). The variable domains of native heavy and light chains each comprise four FR regions, largely a adopting a β-sheet configuration, connected by three CDRs, which form loops connecting, and in some cases forming part of, the β-sheet structure. The CDRs in each chain are held together in close proximity by the FR regions and, with the CDRs from the other chain, contribute to the formation of the antigen-binding site of antibodies. The constant domains are not involved directly in binding an antibody to an antigen, but exhibit various effector functions, such as participation of the antibody in antibody-dependent cellular toxicity.

An antibody that is contemplated for use in the present invention thus can be in any of a variety of forms, including a whole immunoglobulin, an antibody fragment such as Fv, Fab, and similar fragments, a single chain antibody which includes the variable domain complementarity determining regions (CDR), and the like forms, all of which fall under the broad term "antibody", as used herein. The present invention contemplates the use of any specificity of an antibody, polyclonal or monoclonal, and is not limited to antibodies that recognize and immunoreact with a specific antigen. In preferred embodiments, in the context of both the therapeutic and screening methods described below, an antibody or fragment thereof is used that is immuno-specific for an antigen or epitope of the invention.

The term "antibody fragment" refers to a portion of a full-length antibody, generally the antigen binding or variable region. Examples of antibody fragments include Fab, Fab', F(ab')₂ and Fv fragments. Papain digestion of antibodies produces two identical antigen binding fragments, called the Fab fragment, each with a single antigen binding site, and a residual "Fc" fragment, so-called for its ability to crystallize readily. Pepsin treatment yields an F(ab')₂ fragment that has two antigen binding fragments that are capable of cross-linking antigen, and a residual other fragment (which is termed pFc'). Additional fragments can include diabodies, linear antibodies, single-chain antibody molecules, and multispecific antibodies formed from antibody fragments. As used herein, "functional fragment" with respect to antibodies, refers to Fv, F(ab) and F(ab')₂ fragments.

The term "antibody fragment" is used herein interchangeably with the term "antigen binding fragment".

Antibody fragments may be as small as about 4 amino acids, 5 amino acids, 6 amino acids, 7 amino acids, 9 amino acids, about 12 amino acids, about 15 amino acids, about 17 amino acids, about 18 amino acids, about 20 amino acids, about 25 amino acids, about 30 amino acids or more. In general, an antibody fragment of the invention can have any upper size limit so long as it is has similar or immunological properties relative to antibody that binds with specificity to an epitope comprising a peptide sequence selected from any of the sequences identified herein as SEQ ID NOs: 1-3, or a fragment of said sequences. Thus, in context of the present invention the term "antibody fragment" is identical to term "antigen binding fragment".

Antibody fragments retain some ability to selectively bind with its antigen or receptor. Some types of antibody fragments are defined as follows:
(1) Fab is the fragment that contains a monovalent antigen-binding fragment of an antibody molecule. A Fab fragment can be produced by digestion of whole antibody with the enzyme papain to yield an intact light chain and a portion of one heavy chain.
(2) Fab' is the fragment of an antibody molecule can be obtained by treating whole antibody with pepsin, followed by reduction, to yield an intact light chain and a portion of the heavy chain. Two Fab' fragments are obtained per antibody molecule.
   Fab' fragments differ from Fab fragments by the addition of a few residues at the carboxyl terminus of the heavy chain CH1 domain including one or more cysteines from the antibody hinge region.
(3) (Fab')₂ is the fragment of an antibody that can be obtained by treating whole antibody with the enzyme pepsin without subsequent reduction.
(4) F(ab')₂ is a dimer of two Fab' fragments held together by two disulfide bonds.
   Fv is the minimum antibody fragment that contains a complete antigen recognition and binding site. This region consists of a dimer of one heavy and one light chain variable domain in a tight, non-covalent association (V_{H} -V_{L} dimer). It is in this configuration that the three CDRs of each variable domain interact to define an antigen binding site on the surface of the V_{H} -V_{L} dimer. Collectively, the six CDRs confer antigen binding specificity to the antibody. However, even a single variable domain (or half of an Fv comprising only three CDRs specific for an antigen) has the ability to recognize and bind antigen, although at a lower affinity than the entire binding site.
(5) Single chain antibody ("SCA"), defined as a genetically engineered molecule containing the variable region of the light chain, the variable region of the heavy chain, linked by a suitable polypeptide linker as a genetically fused single chain molecule. Such single chain antibodies are also referred to as "single-chain Fv" or "sFv" antibody fragments. Generally, the Fv polypeptide further comprises a polypeptide linker between the VH and VL domains that enables the sFv to form the desired structure for antigen binding. For a review of sFv see Pluckthun in The Pharmacology of Monoclonal Antibodies 113: 269-315 Rosenburg and Moore eds. Springer-Verlag, NY, 1994.

The term "diabodies" refers to a small antibody fragments with two antigen-binding sites, which fragments comprise a heavy chain variable domain (VH) connected to a light chain variable domain (VL) in the same polypeptide chain (VH-VL). By using a linker that is too short to allow pairing between the two domains on the same chain, the domains are forced to pair with the complementary domains of another chain and create two antigen-binding sites. Diabodies are described more fully in, for example, EP 404,097; WO 93/11161, and Hollinger et al., Proc. Natl. Acad Sci. USA 90: 6444-6448 (1993).

The invention contemplate both polyclonal and monoclonal antibody, antigen binding fragments and recombinant proteins thereof which are capable of binding an epitope according to the invention.

The preparation of polyclonal antibodies is well-known to those skilled in the art. See, for example, Green et al. 1992. Production of Polyclonal Antisera, in: Immunochemical Protocols (Manson, ed.), pages 1-5 (Humana Press); Coligan, et al., Production of Polyclonal Antisera in Rabbits, Rats Mice and Hamsters, in: Current Protocols in Immunology, section 2.4.1, which are hereby incorporated by reference.

The preparation of monoclonal antibodies likewise is conventional. See, for example, Kohler & Milstein, Nature, 256:495-7 (1975); Coligan, et al., sections 2.5.1-2.6.7; and Harlow, et al., in: Antibodies: A Laboratory Manual, page 726 ,Cold Spring Harbor Pub. (1988), Monoclonal antibodies can be isolated and purified from hybridoma cultures by a variety of well-established techniques. Such isolation techniques include affinity chromatography with Protein-A Sepharose, size-exclusion chromatography, and ion-exchange chromatography. See, e.g., Coligan, et al., sections 2.7.1-2.7.12 and sections 2.9.1-2.9.3; Barnes, et al., Purification of Immunoglobulin G (IgG). In: Methods in Molecular Biology, 1992, 10:79-104, Humana Press, NY.

Methods of *in vitro* and *in vivo* manipulation of monoclonal antibodies are well known to those skilled in the art. For example, the monoclonal antibodies to be used in accordance with the present invention may be made by the hybridoma method first described by Kohler and Milstein, 1975, Nature 256, 495-7, or may be made by recombinant methods, e.g., as described in US 4,816,567. The monoclonal antibodies for use with the present invention may also be isolated from phage antibody libraries using the techniques described in Clackson et al., 1991, Nature 352: 624-628, as well as in Marks et al., 1991, J Mol Biol 222: 581-597. Another method involves humanizing a monoclonal antibody by recombinant means to generate antibodies containing human specific and recognizable sequences. See, for review, Holmes, et al., 1997, J Immunol 158:2192-2201 and Vaswani, et al., 1998, Annals Allergy, Asthma & Immunol 81:105-115.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional polyclonal antibody preparations that typically include different antibodies directed against different determinants (epitopes), each monoclonal antibody is directed against a single determinant on the antigen. In additional to their specificity, the monoclonal antibodies are advantageous in that they are synthesized by the hybridoma culture, uncontaminated by other immunoglobulins. The modifier "monoclonal" indicates the character of the antibody indicates the character of the antibody as being obtained from a substantially homogeneous population of antibodies, and is not to be construed as requiring production of the antibody by any particular method.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (US 4,816,567); Morrison et al., 1984, Proc Natl Acad Sci 81: 6851-6855.

Methods of making antibody fragments are also known in the art (see for example, Harlow and Lane, Antibodies: A Laboratory Manual, Cold Spring Harbor Laboratory, NY, 1988, incorporated herein by reference). Antibody fragments of the present invention can be prepared by proteolytic hydrolysis of the antibody or by expression in *E. coli* of DNA encoding the fragment. Antibody fragments can be obtained by pepsin or papain digestion of whole antibodies conventional methods. For example, antibody fragments can be produced by enzymatic cleavage of antibodies with pepsin to provide a 5S fragment denoted F(ab')₂. This fragment can be further cleaved using a thiol reducing agent, and optionally a blocking group for the sulfhydryl groups resulting from cleavage of disulfide linkages, to produce 3.5S Fab' monovalent fragments. Alternatively, an enzymatic cleavage using pepsin produces two monovalent Fab' fragments and an Fc fragment directly. These methods are described, for example, in US 4,036,945 and US 4,331,647, and references contained therein. These patents are hereby incorporated in their entireties by reference.

Other methods of cleaving antibodies, such as separation of heavy chains to form monovalent light-heavy chain fragments, further cleavage of fragments, or other enzymatic, chemical, or genetic techniques may also be used, so long as the fragments bind to the antigen that is recognized by the intact antibody. For example, Fv fragments comprise an association of V_{H} and V_{L} chains. This association may be noncovalent or the variable chains can be linked by an intermolecular disulfide bond or cross-linked by chemicals such as glutaraldehyde. Preferably, the Fv fragments comprise V_{H} and V_{L} chains connected by a peptide linker. These single-chain antigen binding proteins (sFv) are prepared by constructing a structural gene comprising DNA sequences encoding the V_{H} and V_{L} domains connected by an oligonucleotide. The structural gene is inserted into an expression vector, which is subsequently introduced into a host cell such as *E. coli.* The recombinant host cells synthesize a single polypeptide chain with a linker peptide bridging the two V domains. Methods for producing sFvs are described, for example, by Whitlow, et al., 1991, In: Methods: A Companion to Methods in Enzymology, 2:97; Bird et al., 1988, Science 242:423-426; US 4,946,778; and Pack, et al., 1993, BioTechnology 11:1271-77. Another form of an antibody fragment is a peptide coding for a single complementarity-determining region (CDR). CDR peptides ("minimal recognition units") are often involved in antigen recognition and binding. CDR peptides can be obtained by cloning or constructing genes encoding the CDR of an antibody of interest. Such genes are prepared, for example, by using the polymerase chain reaction to synthesize the variable region from RNA of antibody-producing cells. See, for example, Larrick, et al., Methods: a Companion to Methods in Enzymology, Vol. 2, page 106 (1991).

The invention contemplates human and humanized forms of non-human (e.g. murine) antibodies. Such humanized antibodies are chimeric immunoglobulins, immunoglobulin chains or fragments thereof (such as Fv, Fab, Fab', F(ab')₂ or other antigen-binding subsequences of antibodies) that contain a minimal sequence derived from non-human immunoglobulin, such as the eitope recognising sequence. For the most part, humanized antibodies are human immunoglobulins (recipient antibody) in which residues from a complementary determining region (CDR) of the recipient are replaced by residues from a CDR of a nonhuman species (donor antibody) such as mouse, rat or rabbit having the desired specificity, affinity and capacity. Humanized antibody(es) containing a minimal sequence(s) of antibody(es) of the invention, such as a sequence(s) recognising an epitope(s) described herein, is one of the preferred embodiments of the invention.

In some instances, Fv framework residues of the human immunoglobulin are replaced by corresponding non-human residues. Furthermore, humanized antibodies may comprise residues that are found neither in the recipient antibody nor in the imported CDR or framework sequences. These modifications are made to further refine and optimize antibody performance. In general, humanized antibodies will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin. For further details, see: Jones et al., 1986, Nature 321, 522-525; Reichmann et al., 1988, Nature 332, 323-329; Presta, 1992, Curr Op Struct Biol 2:593-596; Holmes et al., 1997, J Immunol 158:2192-2201 and Vaswani, et al., 1998, Annals Allergy, Asthma & Immunol 81:105-115.

The generation of antibodies may be achieved by any standard methods of the art for producing polyclonal and monoclonal antibodies using peptide fragments comprising the motifs described above, such as for example fragments of sequence KAEWKSLGEEAWHSK (SEQ ID NO: 1). Such antibodies may be also generated using variants or homologues of peptide sequence of SEQ ID NO: 1. In some embodiments it is preferred to use SEQ ID NO: 1 for the production of the antibody,

The antibodies may also be produced *in vivo* by the individual to be treated, for example, by administering an immunogenic fragment according to the invention to said individual. Accordingly, the present invention further relates to a vaccine comprising an immunogenic fragment described above.

The application also relates to a method for producing an antibody of the invention said method comprising a step of providing of an immunogenic fragment described above.

The invention relates both to 1) an antibody, which is capable of modulating, such as enhancing or attenuating, biological function of human NCAM and/or FGFR and/or FGFR ligands described herein, in particular a function related to cell differentiation and survival, and/or neural plasticity associated with learning and memory, and 2) an antibody, which can recognise and specifically bind to NCAM without modulating biological activity thereof. It is preferred that such antibody is produced by using an immunogenic peptide sequences described above.

The invention relates to use of the described above antibodies for 1) therapeutic applications involving the modulation of activity of FGFR and/or NCAM and/or FGFR ligands described herein; 2) detecting and/or monitoring NCAM polypeptides or fragments thereof in vitro and/or in vivo for diagnostic/prognostic purposes, 4) research purposes.

In one embodiment the invention relates to a pharmaceutical composition comprising an antibody described above.

### 5. Medicament

Cell death plays a key role in normal neuronal development, where 50% of the developing neurons are eliminated through programmed cell death, and in the patho-physiology of neurodegenerative conditions, such as Alzheimer's and Parkinson's diseases. FGFRs and their ligands has been shown to be important determinants of neuronal survival both during development and during adulthood (Reuss and von Bohlen und Halbach (2003) Cell tissue Res, 313:139-57). Therefore, a compound, which is capable to promote neuronal cell survival by binding and activation FGFR is highly desirable. Thus, in one aspect the invention features compounds that promote survival of neural cells and can be used as medicaments for the treatment of conditions involving neural cell death. However, a compound of the invention may also be used as a medicament for promotion of survival of another type of cells, e.g. different type of muscle cells, or, alternatively, for enhancing cell death of still another type of cells, e.g. cancer cells, as the FGFR signalling has been shown to be a survival factor for both muscle and cancer cells (Ozen et al. (2001) J Nat Cancer Inst. 93:1783-90; Miyamoto et al. (1998) J Cell Physiol. 177:58-67; Detilliux et al. (2003) Cardiovasc Res. 57:8-19).

Another approach in the strategy aimed to achieve a compensation for functional cell loss is to create a new pool of said functional cells, for example by commiting the progenitor (stem) cells to differentiate to a new population of differentiated cells, or to initiate regenerating processes in damaged cells. FGFRs play an important role in the mechanisms triggering differentiation of a variety of progenitor cell types (Eswarakumar et al. (2005) Cytokine Growth Factor Rev. 16(2):139-49), cancer cells (St Bernard et al. (2005) Endocrinology 146(3):1145-53) and neural cells (Sapieha et al. (2003) Mol Cell Neurosci. 24(3):656-72).

Activity of cell-surface receptors is strictly regulated in a healthy organism. Mutations, abnormal expression or processing of a receptor or the receptor ligands lead to abnormalities in activity of the receptor and therefore lead to dysfunction of the receptor. The dysfunction of the receptor is in turn a reason for dysfunction of the cells which use the receptor for maintenance of various cellular processes. The latter is the manifestation of a disease. It has also been shown that attenuation of FGFR signalling leads to development of a number of different of pathologic conditions, e.g. diabetes (Hart et al., Nature 2000, 408:864-8). Activation of FGF receptors is involved as in normal, as in pathologic angiogenesis (Slavin, Cell Biol Int 1995, 19:431-44). It is important for development, proliferation, functioning and survival skeletal muscle cells, cardiomyocytes and neurons (Merle at al., J Biol Chem 1995, 270:17361-7; Cheng and Mattson, Neuron 1991, 7:1031-41; Zhu et al., Mech Ageing Dev 1999, 108:77-85). It plays a role in maintenance of normal kidney structure (Cancilla et al., Kidney Int 2001, 60:147-55), and it is implicated in mound healing and cancer disease (Powers et al., Endocr Relat Cancer. 2000, 7:165-97).

Activity of FGFRs has also be reported to be important for stimulation and maintenance of neural plasticity associated with memory and learning (Reuss et al. (2003) Cell Tissue Res. 313(2):139-57; Oomura et al. (1996) Ann N Y Acad Sci. 786:337-47).

The present invention provides compounds capable of modulating the activity of FGFRs, in particular stimulating activity of FGFRs. Consequently, said compounds are concerned by the invention for the production of a medicament for the treatment of diseases, wherein stimulating biological activity dependent on the activity of FGFRs is considered to be beneficial for treatment.

Thus, the medicament of the invention may be used for prevention and/or treatment of
1) diseases and conditions of the central and peripheral nervous system, or of the muscles or of various organs, and/or
2) diseases or conditions of the central and peripheral nervous system, such as postoperative nerve damage, traumatic nerve damage, impaired myelination of nerve fibers, postischaemic damage, e.g. resulting from a stroke, Parkinson's disease, Alzheimer's disease, Huntington's disease, dementias such as multiinfarct dementia, sclerosis, nerve degeneration associated with diabetes mellitus, disorders affecting the circadian clock or neuro-muscular transmission, and schizophrenia, mood disorders, such as manic depression; for treatment of diseases or conditions of the muscles including conditions with impaired function of neuro-muscular connections, such as after organ transplantation, or such as genetic or traumatic atrophic muscle disorders; or for treatment of diseases or conditions of various organs, such as degenerative conditions of the gonads, of the pancreas such as diabetes mellitus type I and II, of the kidney such as nephrosis and of the heart, liver and bowel, and/or
3) postoperative nerve damage, traumatic nerve damage, impaired myelination of nerve fibers, postischaemic, e.g. resulting from a stroke, Parkinson's disease, Alzheimer's disease, Huntington's disease, dementias such as multiinfarct dementia, sclerosis, nerve degeneration associated with diabetes mellitus, disorders affecting the circadian clock or neuro-muscular transmission, and schizophrenia, mood disorders, such as manic depression, and/or
4) cancer disease, and/or
5) prion diseases.

The invention concerns the cancer being any type of solid tumors requiring neoangiogenesis. Cancers of neural system are of particular interest of the invention.

The invention concerns prion diseases selected from the group consisting of scrapie, Creutzfeldt-Jakob disease. It has been shown that FGFRs plays a distinct role in prion diseases (Castelnau et al. (1994) Exp Neurobiol. 130:407-10; Ye and Carp (2002) J Mol Neurosci. 18:179-88).

In another embodiment a compound of the invention may be used for the manufacture of a medicament for
1) promotion of wound-healing, and/or
2) prevention of cell death of heart muscle cells, such as after acute myocardial infarction, or after angiogenesis, and/or
3) revascularisation and/or
4) stimulation of the ability to learn and/or of the short and/or long-term memory.

In still another embodiments a compound of the invention may be used for the manufacture of a medicament for
1) prevention of cell death due to ischemia;
2) prevention of body damages due to alcohol consumption;

In yet still another embodiment a compound of the invention may be used for the manufacture of a medicament for treatment of normal, degenerated or damaged cells which normally in vivo express one or more FGFR low-affinity ligands selected from the group consisting
Neural Cell Adhesion Molecule (NCAM),
   - Neural cell adhesion molecule L1,
   - Neural Cell Adhesion Molecule-2 (NCAM-2),
   - Neuron-glia Cell Adhesion Molecule (Ng-CAM),
   - Neural cell adhesion molecule CALL,
   - Neuroglian,
   - Nr-CAM (HBRAVO, NRCAM, NR-CAM 12),
   - Axonin-1/TAG-1,
   - Axonal-associated Cell Adhesion Molecule (AxCAM),
   - Myelin-Associated Glycoprotein (MAG),
   - Neural cell adhesion molecule BIG-1,
   - Neural cell adhesion molecule BIG-2,
   - Fasciclin (FAS-2),
   - Neural cell adhesion molecule HNB-3/NB-3,
   - Neural cell adhesion molecule HNB-2/NB-2,
   - Cadherin,
   - Junctional Adhesion Molecule-1 (JAM-1),
   - Neural cell adhesion F3/F11 (Contactin),
   - Neurofascin,
   - B-lymphocyte cell adhesion molecule CD22,
   - Neogenin (NEO1),
   - Intercellular Cell Adhesion Molecule-5 (ICAM-5/telencephalin),
   - Galactose binding lectin-12 (galectin-12),
   - Galactose binding lectin-4 (galectin-4),
   - Fibroblast Growth Factor Receptor 1 (FGFR1),
   - Fibroblast Growth Factor Receptor 2 (FGFR2),
   - Fibroblast Growth Factor Receptor 3 (FGFR3),
   - Fibroblast Growth Factor Receptor 4 (FGFR4),
   - Neurotrophin Tyrosin Kinase Type-2 (NTRKT-2),
   - Leukocyte Antigen Related Protein-Tyrosine Phosphatase (LAR-PTPRF),
   - Nephrin,
   - Protein-Tyrosine Phosphatase Receptor type S (PTPRS),
   - Protein-Tyrosine Phosphatase Receptor type kappa (R-PTP-kappa),
   - Protein-Tyrosine Phosphatase Receptor type D (PTPRD),
   - Ephrin type-A receptor 8 (EPHA8/Tyrosine-Protein Kinase Receptor EEK)
   - Ephrin type-A receptor 3 (EPHA8/Tyrosine-Protein Kinase Receptor ETK-1/CEK4),
   - Ephrin type-A receptor 2,
   - Insulin Receptor (IR),
   - Insulin-like Growth Factor-1 Receptor (IGF-1),
   - Insulin-related Receptor (IRR),
   - Tyrosine-Protein Kinase Receptor Tie-1,
   - Roundabout receptor-1 (robo-1),
   - Neuronal nicotinic acetylcholine receptor alpha 3 subunit (CHRNA3),
   - Neuronal acetylcholine receptor alpha 6 subunit,
   - Platelet-Derived Growth Factor Receptor Beta (PDGFRB),
   - Interleukin-6 Receptor (IL-6R),
   - Interleukin-23 Receptor (IL-23R),
   - Beta-common cytokine receptor of IL-3, IL5 and GmCsf,
   - Cytokine Receptor-Like molecule 3 (CRLF1),
   - Class I Cytokine Receptor (ZCYTOR5),
   - Netrin-1 receptor DCC,
   - Leukocyte Fc Receptor-like Protein (IFGP2),
   - Macrophage Scavenger Receptor 2 (MSR2),
   - Granulocyte Colony Stimulating Factor Receptor,
   - perlecan,
   - ADAM-19,
   - ADAM-8,
   - ADAM-12,
   - ADAM-28,
   - ADAM-33,
   - ADAM-9,
   - ADAM-7,
   - ADAM-1A Fertilin alpha,
   - ADAM-15,
   - Metalloproteinase-desintegrin domain containing protein (TECAM),
   - Metalloproteinase 1,
   - Collagen type VII, I,
   - Fibronectin,
   - Tenascin-R,
   - Cytokine-like factor-1 (CLF-1).

According to invention the above listed proteins are natural low-affinity ligands of FGFR and a compound of the invention comprising at least one peptide fragment of the invention capable of binding to FGFR and activating FGFR signalling, may advantageously be used as a mimic of any of the above proteins in conditions wherein the function of said proteins is impaired. In particular, such compound is useful for treatment normal, degenerated or damaged NCAM presenting cells.

A medicament according to the invention comprises an effective amount of one or more of peptide sequences or compounds as defined above, or a pharmaceutical composition comprising one or more compound of the invention and pharmaceutically acceptable additives.

Biological activity associated with specific structural features of the peptide sequences of the invention (discussed above) should be taken in the account when manufacturing a medicament or preparing a pharmaceutical composition. In some embodiments it may be preferred to use sequence KAEWKSLGEEAWHSK (SEQ ID NO: 1) for the manufacturing a medicament, in another embodiment sequence SIDRVEPYSSTAQVQFD (SEQ ID NO: 2) may be preferred, still in other embodiments it may be preferred to use a compound comprising both sequences, or any other type of compounds discussed in the application. Accordingly, in one preferred embodiment a medicament and/or pharmaceutical composition of the invention may be for stimulating of learning and memory, in another embodiment a pharmaceutical composition may be for stimulating neuronal survival, in still another embodiment a pharmaceutical composition may be for stimulating neural cell differentiation, in yet another embodiment a pharmaceutical composition may be i) for stimulating memory and learning and stimulating cell survival, or ii) for stimulating memory and learning and stimulating neurite outgrowth, or iii) stimulating neurite outgrowth and cell survival. When concerned treatment of a condition or disease wherein a FGFR ligand of above plays a role, a pharmaceutical composition may be preferably used for enhancing or attenuating a function/biological activity of said ligand, which may be different from the biological activities mentioned above.

The invention also relates to a medicament and pharmaceutical composition comprising an antibody of the invention as described above.

A further aspect of the invention is a process of producing a pharmaceutical composition, comprising mixing an effective amount of one or more of the compounds of the invention, or a pharmaceutical composition according to the invention with one or more pharmaceutically acceptable additives or carriers.

In one embodiment of the process as mentioned above, the compounds are used in combination with a prosthetic device, wherein the device is a prosthetic nerve guide. Thus, in a further aspect, the present invention relates to a prosthetic nerve guide, characterised in that it comprises one or more of the compounds or the pharmaceutical composition as defined above. Nerve guides are known in the art.

The invention relates to the use a medicament and/or pharmaceutical composition comprising the compound of invention for the treatment or prophylaxis of any of the diseases and conditions mentioned below.

Such medicament and/or pharmaceutical composition may suitably be formulated for oral, percutaneous, intramuscular, intravenous, intracranial, intrathecal, intracerebroventricular, intranasal or pulmonal administration.

Strategies in formulation development of medicaments and compositions based on the compounds of the present invention generally correspond to formulation strategies for any other protein-based drug product. Potential problems and the guidance required to overcome these problems are dealt with in several textbooks, e.g. "Therapeutic Peptides and Protein Formulation. Processing and Delivery Systems", Ed. A.K. Banga, Technomic Publishing AG, Basel, 1995.

Injectables are usually prepared either as liquid solutions or suspensions, solid forms suitable for solution in, or suspension in, liquid prior to injection. The preparation may also be emulsified. The active ingredient is often mixed with excipients which are pharmaceutically acceptable and compatible with the active ingredient. Suitable excipients are, for example, water, saline, dextrose, glycerol, ethanol or the like, and combinations thereof. In addition, if desired, the preparation may contain minor amounts of auxiliary substances such as wetting or emulsifying agents, pH-buffering agents, or which enhance the effectiveness or transportation of the preparation.

Formulations of the compounds of the invention can be prepared by techniques known to the person skilled in the art. The formulations may contain pharmaceutically acceptable carriers and excipients including microspheres, liposomes, microcapsules, nanoparticles or the like.

The preparation may suitably be administered by injection, optionally at the site, where the active ingredient is to exert its effect. Additional formulations which are suitable for other modes of administration include suppositories, nasal, pulmonal and, in some cases, oral formulations. For suppositories, traditional binders and carriers include polyalkylene glycols or triglycerides. Such suppositories may be formed from mixtures containing the active ingredient(s) in the range of from 0.5% to 10%, preferably 1-2%. Oral formulations include such normally employed excipients as, for example, pharmaceutical grades of mannitol, lactose, starch, magnesium stearate, sodium saccharine, cellulose, magnesium carbonate, and the like. These compositions take the form of solutions, suspensions, tablets, pills, capsules, sustained release formulations or powders and generally contain 10-95% of the active ingredient(s), preferably 25-70%.

Other formulations are such suitable for nasal and pulmonal administration, e.g. inhalators and aerosols. '

The active compound may be formulated as neutral or salt forms. Pharmaceutically acceptable salts include acid addition salts (formed with the free amino groups of the peptide compound) and which are formed with inorganic acids such as, for example, hydrochloric or phosphoric acids, or such organic acids as acetic acid, oxalic acid, tartaric acid, mandelic acid, and the like. Salts formed with the free carboxyl group may also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

The preparations are administered in a manner compatible with the dosage formulation, and in such amount as will be therapeutically effective. The quantity to be administered depends on the subject to be treated, including, e.g. the weight and age of the subject, the disease to be treated and the stage of disease. Suitable dosage ranges are per kilo body weight normally of the order of several hundred µg active ingredient per administration with a preferred range of from about 0.1 µg to 5000 µg per kilo body weight. Using monomeric forms of the compounds, the suitable dosages are often in the range of from 0.1 µg to 5000 µg per kilo body weight, such as in the range of from about 0.1 µg to 3000 µg per kilo body weight, and especially in the range of from about 0.1 µg to 1000 µg per kilo body weight. Using multimeric forms of the compounds, the suitable dosages are often in the range of from 0.1 µg to 1000 µg per kilo body weight, such as in the range of from about 0.1 µg to 750 µg per kilo body weight, and especially in the range of from about 0.1 µg to 500 µg per kilo body weight such as in the range of from about 0.1 µg to 250 µg per kilo body weight. In particular when administering nasally smaller dosages are used than when administering by other routes. Administration may be performed once or may be followed by subsequent administrations. The dosage will also depend on the route of administration and will vary with the age and weight of the subject to be treated. A preferred dosage of multimeric forms would be in the interval 1 mg to 70 mg per 70 kg body weight.

For some indications a localised or substantially localised application is preferred.

For another application, intranasal application is preferred.

Some of the compounds of the present invention are sufficiently active, but for some of the others, the effect will be enhanced if the preparation further comprises pharmaceutically acceptable additives and/or carriers. Such additives and carriers will be known in the art. In some cases, it will be advantageous to include a compound, which promote delivery of the active substance to its target.

In many instances, it will be necessary to administrate the formulation multiple times. Administration may be a continuous infusion, such as intraventricular infusion or administration in more doses such as more times a day, daily, more times a week, weekly, etc. It is preferred that administration of the medicament is initiated before or shortly after the individual has been subjected to the factor(s) that may lead to cell death. Preferably the medicament is administered within 8 hours from the factor onset, such as within 5 hours from the factor onset. Many of the compounds exhibit a long term effect whereby administration of the compounds may be conducted with long intervals, such as 1 week or 2 weeks.

In connection with the use in nerve guides, the administration may be continuous or in small portions based upon controlled release of the active compound(s). Furthermore, precursors may be used to control the rate of release and/or site of release. Other kinds of implants and well as oral administration may similarly be based upon controlled release and/or the use of precursors.

### 6. Treatment

Compounds/compositions for use in medical treatment according to the invention are in one embodiment useful for inducing differentiation, modulating proliferation, stimulating regeneration, neuronal plasticity and survival of cells, for example cells being implanted or transplanted. This is particularly useful when using compounds having a long term effect.

In further embodiment the treatment as defined above may be for stimulation of survival of cells which are at risk of dying due to a variety of factors, such as traumas and injuries, acute diseases, chronic diseases and/or disorders, in particular degenerative diseases normally leading to cell death, other external factors, such as medical and/or surgical treatments and/or diagnostic methods that may cause formation of free radicals or otherwise have cytotoxic effects, such as X-rays and chemotherapy. In relation to chemotherapy the FGFR binding compounds according to the invention are useful in cancer treatment of all cancer cells presenting FGFRs.

Thus, the treatment comprises treatment and/or prophylaxis of cell death in relation to diseases or conditions of the central and peripheral nervous system, such as postoperative nerve damage, traumatic nerve damage, e.g. resulting from spinal cord injury, impaired myelination of nerve fibers, postischaemic damage, e.g. resulting from a stroke, multiinfarct dementia, multiple sclerosis, nerve degeneration associated with diabetes mellitus, neuro-muscular degeneration, schizophrenia, Alzheimer's disease, Parkinson's disease, or Huntington's disease.

Also, in relation to diseases or conditions of the muscles including conditions with impaired function of neuro-muscular connections, such as genetic or traumatic atrophic muscle disorders; or for the treatment of diseases or conditions of various organs, such as degenerative conditions of the gonads, of the pancreas, such as diabetes mellitus type I and II, of the kidney, such as nephrosis the compounds according to the invention may be used for inducing differentiation, modulating proliferation, stimulate regeneration, neuronal plasticity and survival , i.e. stimulating survival.

Furthermore, the compound and/or pharmaceutical composition may be for preventing cell death of heart muscle cells, such as after acute myocardial infarction, in order to induce angiogenesis. Furthermore, in one embodiment the compound and/or pharmaceutical composition is for the stimulation of the survival of heart muscle cells, such as survival after acute myocardial infarction. In another aspect the compound and/or pharmaceutical composition is for revascularisation, such as after injuries.

The compound and/or pharmaceutical composition for use for the promotion of wound-healing is also within the scope of the invention. The present compounds are capable of stimulating angiogenesis and thereby promote the wound healing process.

The invention further discloses the compound and/or pharmaceutical composition for use in the treatment of cancer. Regulation of activation of FGFR is important for tumor agiogenesis, proliferation and spreading.

In yet a further embodiment of the compound and/or pharmaceutical composition for use for the stimulation of the ability to learn and/or of the short and/or long term memory is disclosed as FGFR activity is important for differentiation of neural cells.

In still another embodiment a compound and/or pharmaceutical composition of the invention is for treatment of body damages due to alcohol consumption. Developmental malformations of foetuses, long-term neurobehavioral alterations, alcoholic liver disease is in particular concerned.

The compound and/or pharmaceutical composition for use in the treatment of prior disease is still another embodiment of the invention.

In particular the compound and/or pharmaceutical composition of the invention may be used in the treatment of clinical conditions, such as neoplasma such as malignant neoplasms, benign neoplasms, carcinomas in situ and neoplasms of uncertain behavior, diseases of endocrine glands, such as diabetes mellitus, psychoses, such as senile and presenile organic psychotic conditions, alcoholic psychoses, drug psychoses, transient organic psychotic conditions, Alzheimer's disease, cerebral lipidoses, epilepsy, general paresis [syphilis], hepatolenticular degeneration, Huntington's chorea, Jakob-Creutzfeldt disease, multiple sclerosis, Pick's disease of the brain, syphilis,Schizophrenic disorders, affective psychoses, neurotic disorders, personality disorders, including character neurosis, nonpsychotic personality disorder associated with organic brain syndromes, paranoid personality disorder, fanatic personality, paranoid personality (disorder), paranoid traits, sexual deviations and disorders, mental retardation, disease in the nervesystem and sense organs, cognitive anomalies, inflammatory disease of the central nervous system, such as meningitis, encephalitis,Cerebral degenerations such as Alzheimer's disease, Pick's disease, senile degeneration of brain, communicating hydrocephalus, obstructive hydrocephalus, Parkinson's disease including other extra pyramidal disease and abnormal movement disorders, spinocerebellar disease, cerebellar ataxia, Marie's,Sanger-Brown, Dyssynergia cerebellaris myoclonica, primary cerebellar degeneration, such as spinal muscular atrophy, familial, juvenile, adult spinal muscular atrophy, motor neuron disease, amyotrophic lateral sclerosis, motor neuron disease, progressive bulbar palsy, pseudobulbar palsy, primary lateral sclerosis, other anterior horn cell diseases, anterior horn cell disease, unspecified, other diseases of spinal cord, syringomyelia and syringobulbia, vascular myelopathies, acute infarction of spinal cord (embolic) (nonembolic), arterial thrombosis of spinal cord, edema of spinal cord, subacute necrotic myelopathy, subacute combined degeneration of spinal cord in diseases classified elsewhere, myelopathy, drug-induced, radiation-induced myelitis, disorders of the autonomic nervous system, disorders of peripheral autonomic, sympathetic, parasympathetic, or vegetative system, familial dysautonomia [Riley-Day syndrome], idiopathic peripheral autonomic neuropathy, carotid sinus syncope or syndrome, cervical sympathetic dystrophy or paralysis. peripheral autonomic neuropathy in disorders classified elsewhere, amyloidosis, diseases of the peripheral nerve system, brachial plexus lesions, cervical rib syndrome, costoclavicular syndrome, scalenus anterior syndrome, thoracic outlet syndrome, brachial neuritis or radiculitis, including in newborn. Inflammatory and toxic neuropathy, including acute infective polyneuritis, Guillain-Barre syndrome, Postinfectious polyneuritis, polyneuropathy in collagen vascular disease, disorders affecting multiple structures of eye, purulent endophthalmitis, diseases of the ear and mastoid process, chronic rheumatic heart disease, ischaemic heart disease, arrhythmia, diseases in the pulmonary system, abnormality of organs and soft tissues in newborn, including in the nerve system, complications of the administration of anesthetic or other sedation in labor and delivery, diseases in the skin including infection, insufficient circulation problem, injuries, including after surgery, crushing injury, burns. Injuries to nerves and spinal cord, including division of nerve, lesion in continuity (with or without open wound), traumatic neuroma (with or without open wound), traumatic transient paralysis (with or without open wound), accidental puncture or laceration during medical procedure, injury to optic nerve and pathways, optic nerve injury, second cranial nerve, injury to optic chiasm, injury to optic pathways, injury to visual cortex, unspecified blindness, injury to other cranial nerve(s), injury to other and unspecified nerves. Poisoning by drugs, medicinal and biological substances, genetic or traumatic atrophic muscle disorders; or for the treatment of diseases or conditions of various organs, such as degenerative conditions of the gonads, of the pancreas, such as diabetes mellitus type I and II, of the kidney, such as nephrosis. Scrapie, Creutzfeldt-Jakob disease, Gerstmann-Straussler-Sheinker (GSS) disease.

The treatment and/or prevention of the above conditions and symptoms comprises a step of administering an effective amount of a compound and/or pharmaceutical composition of the invention to an individual in need.

### Examples

The peptide fragments of NCAM TIMGLKPETRYAVR (SEQ ID NO: 5), termed herein as the EFL peptide, and EVYVVAENQQGKSKA (SEQ ID NO: 4), termed herein as the FGL peptide, have been used in the below experiments as positive controls.

Sequence KAEWKSLGEEAWHSK (SEQ ID NO: 1) is termed herein as the CDL peptide and sequence SIDRVEPYSSTAQVQFD (SEQ ID NO: 2) is termed herein as the ABL peptide.

### Example 1. Stimulation of neurite outgrowth

Cerebellar granule neurons (CGN) were prepared from postnatal day seven Wistar rats largely as previously described (Neiiendam et al, (2004) J Neurochem. 91 (4):920-35). Cerebellar tissue was dissected in modified Krebs-Ringer solution kept on ice, and treated as described for the hippocampal neurons above. All cell cultures were incubated at 37°C in a humidified atmosphere containing 5 % CO₂. All animals were handled in accordance with the national guidelines for animal welfare.

Dissociated cells were plated at a density of 10,000 cells/cm² on uncoated 8-well permanox Lab-Tek chamber slides in Neurobasal medium supplemented with 0.4 % (w/v) bovine serum albumin (BSA; Sigma-Aldrich), 2 % (v/v) B27 Neurobasal supplement, 1 % (v/v) glutamax, 100 U/ml penicillin, 100 µg/ml streptomycin and 2 % 1 M HEPES (all from Gibco, BRL). Peptide solutions without or with inhibitors of various signal transduction pathways were added to a total volume of 300 µl/cm², and the slides were incubated at 37°C. After 24 hours, the neurons were fixed with 4 % (v/v) formaldehyde for 20 minutes and thereafter immunostained using primary rabbit antibodies against GAP-43 and Alexa Fluor secondary goat anti-rabbit antibodies. Images of at least 200 neurons for each group in each individual experiment were obtained systematically by using computer assisted fluorescence microscopy as previously described (Rønn et al., 2000 op. cit.). Briefly, a Nikon Diaphot inverted microscope with a Nikon Plan 20x objective (Nikon, Tokyo, Japan) coupled to a video camera (Grundig Electronics, Germany) was used for recordings. The same software package as described above for the dopaminergic neurite outgrowth assay was used to process the recorded images.

As it appears from Figure 1 (A and B), the EFL and CDL peptides of NCAM F3, 1 are strong stimulators of neurite outgrowth neurons grown in culture without substrate, whereas the ABL peptide is inactive in these culture conditions. The effect of the EFL peptide can be abolished by application of SU 5402, a known inhibitor of FGFR, indicating that the stimulation is dependent on activation of the receptor.

### Example 2. Stimulation of survival of neurons

### Survival assay

Primary cultures of CGN were plated at a density of 100,000 cells/cm² on poly-L-lysine coated 8-well permanox slides in Neurobasal-A medium (Gibco, BRL) supplemented with 2 % (v/v) B27, 0.5 % (v/v) glutamax, 100 U/ml penicillin, 100 µg/ml streptomycin and KCl, making the final concentration of KCI in the medium 40 mM. 24 hours after plating, cytosine-β-D-arabinofuranoside (Ara-C; Sigma-Aldrich) was added to a final concentration of 10 µM to avoid proliferation of glial cells, after which the neurons were allowed to differentiate for further six days at 37°C. Apoptotic cell death was induced by washing twice and changing the medium to Basal Medium Eagle (BME; Gibco BRL) supplemented with 1 % (v/v) glutamine, 100 U/ml penicillin and 100 µg/ml streptomycin, 3.5 g D-glucose/I and 1 % (v/v) sodium pyruvate (Gibco BRL) together with various concentrations of peptide. Thereby the concentration of potassium in the cultures was reduced to 5 mM KCI. Two days after induction of apoptosis, the cells were fixed with 4 % formaldehyde and stained with Hoechst 33258 as described for the survival assay employing hippocampal neurons.

The assay is performed as described by D'Mello et al., (1993) (Proc Natl Acad Sci U S A. 90:10989-93).

### Caspase-3-like activity assay

Primary cultures of CGN were prepared as described above and apoptosis in cells was induced as described above.

For the caspase-3 assay, cultured CGN were dislodged, collected by centrifugation and lyzed. Aliquots of lysed cells were used for the assay which was performed according to the manufacturer's instructions (Promega, USA),

### Results

From Figure 2 (A and B) it appears that the CDL and EFL peptides (dendrimers) (Figure 2A and 2B correspondingly) significantly promote survival of CGN in culture, and the effect is higher then the effect of well-known cell survival promoters such as the C3 peptide (Berezin V and Bock E. (2004) J Mol Neurosci 22(1-2):33-39) and GDNF (Krieglstein K (2004) Cell Tissue Res 318(1):73-80) (Figure 2A). The ABL peptide does not influence survival of CGN neurons in culture.

Figure 3 demonstrates that the CDL and EFL peptides inhibit the apoptosis-associated Caspase-3-like activity in cells maintained in culture mediun depleted of KCI and that the effect is comparable with the effect of high KCI (40 mM).

### Example 4. Binding of fragments of NCAM F3,1 to FGFR1

Different peptide fragments representing all "strand-loop-strand" structural domains of NCAM F3,1 (AB - A strand-loop-B strand (corresponds to the ABL peptide SEQ ID NO: 2); CD - C strand-loop-D strand (corresponds to the CDL peptide SEQ ID NO: 1); EF - E strand-loop-F strand (corresponds to the EFL peptide SEQ ID NO: 5); BC - B strand-loop-C strand; DE - D strand-loop-E strand; FG - F strand-loop-G strand) have been prepared synthetically.

The FGFR Ig module 3 and modules 2, 3 were expressed in Drosophila S2 cells (Invitrogen, USA) according to the manufacturer's instructions. All the proteins were purified by affinity chromatography using Ni²⁺-NTA resin (Qiagen, USA) and/or ion exchange chromatography and gel filtration.

Binding analysis was performed using a BIAcoreX instrument (Biosensor AB) at 25 °C using 10 mM sodium phosphate pH 7.4, 150 mM NaCl as running buffer. The flow-rate was 5 µl/min. Data were analysed by non-linear curve-fitting using the manufacture's software. The FGFR Ig modules 2, 3 were immobilized on a sensor chip CM5 using amine coupling kit (Biosensor AB) as follows: 1) the two halves of the chip (designated Fc1 and Fc2) were activated by 20 µl activation solution; 2) the protein was immobilized on Fc1 using 12 µl 20 µg/ml protein in 10 mM sodium phosphate buffer pH 6.0; 3) Fc1 and Fc2 were blocked by 35 µl blocking solution. Binding of various compounds to the immobilized FGFR modules was studied as follows: A compound was injected simultaneously into Fc1 (with the immobilized FGFR modules) and Fc2 (with nothing immobilized). The curve representing unspecific binding of the compound to the surface of Fc2 was subtracted from the curve representing binding of the protein to the immobilized FGFR modules and the surface of Fc1. The resulting curve was used for analysis. The results of the binding experiments are present on Figure 4. From Figure 4 it appears that the ABL, CDL and EFL peptides of NCAM F3, 1 are capable of direct binding to FGFR.

### Example 5. Phosphorylation of FGFR

TREX-293 cells (Invitrogen) were stably transfected with human FGFR1 having a C-terminal Strepll-tag (IBA biotech), using the Flp-In system (Invitrogen). For the study of phosphorylation: ~2x10⁷ cells were starved overnight before stimulation for 20 min with the specified compounds. Cells were lysed in PBS with 1% NP-40 and phosphatase inhibitors cocktail set II (Calbiochem). The cleared cell lysates were incubated with 50 µl agarose-coupled anti-phosphotyrosine antibodies (4G10-AC, Upstate Biotechnologies) for 3 hrs at 4°C. Care was taken to calibrate the amount of cells employed, so that considerable increases in phosphorylated FGFR could be detected. The bound protein was washed, eluted using 150 mM phenyl phosphate (Sigma), precipitated by 12% trichloroacetic acid, washed in cold acetone and dissolved in SDS-PAGE sample buffer. Immunoblotting was performed using antibodies against the recombinant Strepll-tag (IBA biotech).

As appears from Figure 5, the ABL, CDL and EFL peptides increase (2-3 fold) phosphorylation of FGFR compared to non-stimulated cells.

### Example 6. Learning and memory paradigms

### The Contextual Fear Conditioning test (CFC)

The rats were handled for 120 seconds every day for 3 days preceding the first day of the experiment. On day 1 (training), the rats received a 1-second shock (unconditioned stimuli) each minute for two minutes. Animals were injected immediately after training with 5 µl EFL, ABL, CDL or control peptide, or the vehicle. Each treatment group consisted of 11-14 rats. In total, 39 animals were evaluated. Animals were tested on days 2, 7 and 30 by placing them in the chamber used for conditioning, but in the absence of a shock, for 8 minutes. Each rat was rated as either "freezing" or "active", every 2 seconds, using a time-sampling procedure.

An increase in time of freezing after administration of the peptides was detected when animals were tested on day 1 (from 40.0% to approximately 50.3% (EFL)) and day 8 (from 32% to 42.5% (CDL and EFL)) after conditioning compared to rats receiving placebo or the control. This indicates that the EFL and CDL peptides are capable of improving the long-term retention of the conditioned fear response when administered to post-training in adult male Wistar rats.

### Social Recognition test (SRT)

The animals were evaluated using the SRT, 1 hour and 24 hours after subcutaneous injection of the peptide or vehicle.

*Habituation session:* On the day before the social recognition test (SRT), one habituation session was performed under similar conditions as the test session for adaptation to the experimental room, time when the test was performed, test cage, and contact with juvenile rats (a different juvenile was used during habituation session and social recognition test). The inter-trial interval for the habituation session was of 1 hour.

*Experimental session:* Each experimental session consisted of an initial and a test trial separated by an inter-trial (retention) interval of 2 hours.

*Initial trial.* Adult animals were individually housed 1 hour before testing in the test cage. For testing, a juvenile was placed into the cage and the social investigation by the adult rat was measured cumulatively for 4 min. Sniffing and grooming of body parts, anogenital sniffing and close following were scored. Behaviour records were obtained by recording with a digital video camera and saved directly on digital video disks (dvd). During the 2 hours retention time, animals remained singly housed. Juveniles were likewise individually housed 1 h prior to testing of the adult and remained singly housed during the 2 hours retention time.

*Test trial.* The retest was performed after a delay of 2 hours under essentially the same experimental conditions as the initial trial. The test was conducted with an unfamiliar juvenile, thus each adult was exposed to a juvenile they had not previously experienced.

Juvenile recognition was determined to be present during the second exposure, if the adult spend significantly less time investigating the previously exposed juvenile. A lack of difference between the times spent investigating the juvenile during the first and second exposures, indicated that the adult failed to recognize the familiar juvenile.

Further, the recognition ratio (RR): T₂/(T₁+T₂), T₁ and T₂ being the time spent on investigating the juvenile animal during the first (initial) and the second (test) trial respectively (Kogan et al., 2000) was used as an index of social recognition memory. If the adult rat demonstrated aggressive, passive (investigating time less than 25% of total exposure time) or sexual behaviour towards the juvenile rat, the rat was not scored.

### Data Analysis and Statistical calculations

The data obtained (T₁ and T₂) were carefully entered into a computerised database (GRAPH PAD version 4). The following parameters were analysed:
1) Duration of T₁ and T₂:
   a) A significant difference between T₁ and T₂ of a same animal (obtained using paired t-test analysis) was taken as an indication of presence of social memory.
   b) A significant difference between T₁ of different treatments groups, (obtained by unpaired t-test in case of comparison between 2 groups, or by one-way ANOVA followed by Neuman-Keuls post-hoc test in case of comparison between more than 2 groups), was taken as an indication that a treatment was affecting the investigative behaviour of the animals.
2) Social Recognition Ratio (RR): It was estimated as T₂/(T₁+T₂), T₁ and T₂ being the time spent investigating the juvenile animal during the first and the second meeting, respectively. The evaluation of the RR was useful as a way of normalising the data obtained in different tests.
   a) A significant difference between RR of different treatments groups and the theoretical value of 0.5 (obtained by one sample t-test) was taken as an indication of presence of social memory.
   b) A significant difference between RR of different treatments groups (obtained by unpaired t-test in case of comparison between 2 groups, or by one-way ANOVA followed by Neuman-Keuls post-hoc test in case of comparison between more than 2 groups) was taken as an indication that a treatment was affecting social memory.
   c) A significant difference between RR of different treatments groups (obtained by unpaired t-test), when the animals were exposed to a familiar juvenile versus when they were exposed to a novel juvenile was taken as an indication that the effect seen with the familiar juvenile was specific to cognition.

The test peptides (ABL and EFL) or vehicle (water) were administrated subcutaneously at a concentration of 4 mg/kg (2mg/ml).

Results of SRT are shown on Figure 6. The CDL and EFL peptides shows significant differences between T₁ and T₂ of the same animal (obtained using paired t-test analysis), indicating the presence of social memory.

### Statistical analysis

The results obtained were expressed as the arithmetic means ± SEM. The statistical assessment of the peptides effects were carried out using one-way ANOVA on CFC or SRT of the three different groups of rats followed by the least significance difference (LSD) test, when values of ANOVA reached *P* ≤0.05. ANOVA with replication was used for the assessment of specificity of the STR procedure by comparison of the results of rat groups after known JR vs unknown JR presentation as the repeated measure. The statistical analysis of the peptide effect on CFC or social memory was carried out by using one-way ANOVA.

## Claims

1. A peptide of 11 to 18 amino acid residues comprising the amino acid motif of the formula (I)
K/R-x^{p}-D/E/N/Q-x^{p}-x₀-S-x₁-x₂-x₃-D/E/N/Q-x^{p}
wherein
x^{p} is independently selected from A,P,V or W,
x₀ is K, R or Y, and
x₁, x₂ and x₃ are independently any amino acid residue.

2. The peptide according to claim 1, wherein x^{p} is selected from A, V or W.

3. The peptide according to claim 2, wherein the amino acid motif is
K/R-V/A-D/E/N/Q-W-x₀-S-x₁-x₂-x₃-D/E/N/Q-V/A,
wherein x₀, x₁, x₂ and x₃ are as defined in claim 1.

4. The peptide according to claim 1, wherein x^{p} is selected from A, V or P.

5. The peptide according to claim 4, wherein the amino acid motif is
K/R-V/A-D/E/N/Q-P-x₀-S-x₁-x₂-x₃-D/E/N/Q-V/A,
wherein x₀, x₁, x₂ and x₃ are as defined in claim 1.

6. The peptide according to any of the claims 1 to 5, wherein x₁, x₂ and x₃ are independently selected from A, G, L, S, T, or E.

7. The peptide according to claim 6, wherein the peptide sequence has a length of 11 to 14 amino acid residues.

8. The peptide according to claim 6, wherein the peptide sequence has a length of 15 to 18 amino acid residues.

9. The peptide according to claim 8, wherein the peptide sequence is KAEWKSLGEEAWHSK (SEQ ID NO: 1).

10. The peptide according to claim 8, wherein the peptide sequence is SIDRVNPYSSTAQVQFD (SEQ ID NO: 3).

11. The peptide according to any of the preceding claims 1-10, wherein said peptide is formulated as a multimeric compound comprising two or more copies of said individual peptide sequence.

12. The peptide according to claim 11, wherein the multimeric compound is a dimer comprising two identical copies of an individual peptide sequence according any of the claims 1 to 10.

13. The peptide according to claim 11, wherein the multimeric compound is a dimer comprising two different peptide sequences according to claims 1 to 10.

14. The peptide according to claim 11, wherein the multimeric compound is a dimer comprising a peptide according to any of the claims 1 to 10 and a peptide selected from EVYVVAENQQGKSKA (SEQ ID NO: 4) or TIMGLKPETRYAVR (SEQ ID NO: 5).

15. The peptide according claim 11, wherein the multimeric compound is a dendrimer comprising four identical copies of a peptide according to any of the claims 1 to 10 linked to a backbone structure of three lysine residues.

16. The peptide according to any of the preceding claims, wherein the peptide fragment is capable of binding and activating a receptor of the fibroblast growth factor receptor (FGFR) family comprising fibroblast growth factor receptor-1 (FGFR-1), fibroblast growth factor receptor-2 (FGFR-2), fibroblast growth factor receptor-3 (FGFR-3), fibroblast growth factor receptor-4 (FGFR-4) and fibroblast growth factor receptor-5 (FGFR-5).

17. The peptide according to claim 16, wherein the receptor is FGFR-1.

18. The peptide according to any of the preceding claims, said peptide is capable of stimulating learning and memory.

19. A peptide of 11 to 18 amino acid residues comprising the amino acid motif of the formula (I)
K/R-x^{P}-D/E/N/Q-x^{P}-x₀-S-x₁-x₂-x₃-D/E/N/Q-x^{P}
wherein
x^{P} is independently A, P, V or W,
x₀ is K, R or Y, and
x₁, x₂ and x₃ are independently any amino acid residue,
for use in stimulating memory and learning.

20. The peptide according to claim 19, wherein x^{P} is selected from A, V or W.

21. The peptide according to claim 20, wherein the amino acid motif is
K/R-V/A-D/E/N/Q-W-x₀-S-x₁-x₂-x₃-D/E/N/Q-V/A,
wherein x₀, x₁, x₂ and x₃ are as defined in claim 1.

22. The peptide according to claim 19, wherein x^{P} is selected from A, V or P.

23. The peptide according to claim 22, wherein the amino acid motif is
K/R-WA-D/E/N/Q-P-x₀-S-x₁-x₂-x₃-D/E/N/Q-V/A,
wherein x₀, x₁, x₂ and x₃ are as defined in claim 1.

24. The peptide according to any of the claims 19 to 23, wherein x₁, x₂ and x₃ are independently selected from A, G, L, S, T, or E.

25. The peptide according to claim 24, wherein the peptide sequence has the length of 11 to 14 amino acid residues.

26. The peptide according to claim 24, wherein the peptide sequence has the length of 15 to 18 amino acid residues.

27. The peptide according to claim 26, wherein the peptide sequence is KAEWKSLGEEAWHSK (SEQ ID NO:1).

28. The peptide according to claim 26, wherein the peptide sequence is SIDRVEPYSSTAQVQFD (SEQ ID NO: 2).

29. The peptide according to claim 26, wherein the peptide sequence is SIDRVNPYSSTAQVQFD (SEQ ID NO: 3).

30. The peptide according to any of the preceding claims 19 to 29, wherein a peptide sequence according to any of the claims 1 to 18 is formulated as a multimeric compound comprising two or more copies of said individual peptide sequence.

31. The peptide according to claim 30, wherein the multimeric compound is a dimer comprising two identical copies of an individual peptide sequence according any of the claims 1 to 18.

32. The peptide according to claim 30, wherein the multimeric compound is a dimer comprising two different peptide sequences according to claims 1 to 18.

33. The peptide according to claim 30, wherein the multimeric compound is a dimer comprising a sequence according to any of the claims 1 to 18 and a sequence selected from TIMGLKPETRYAVR (SEQ ID NO: 5) or EVYVVAENQQGKSKA (SEQ ID NO: 4).

34. The peptide according to claim 30, wherein the multimeric compound is a dendrimer comprising four identical copies of a peptide sequence according to any of the claims 1 to 18 linked to a backbone structure of three lysine residues.

35. The peptide according to any of the preceding claims 19 to 34, wherein the peptide fragment is capable of binding and activating a receptor of the fibroblast growth factor receptor (FGFR) family comprising fibroblast growth factor receptor-1 (FGFR-1), fibroblast growth factor receptor-2 (FGFR-2), fibroblast growth factor receptor-3 (FGFR-3), fibroblast growth factor receptor-4 (FGFR-4) and fibroblast growth factor receptor-5 (FGFR-5).

36. The peptide according to claim 35, wherein the receptor is FGFR-1.

37. A peptide of 11 to 18 amino acid residues comprising the amino acid sequence of the formula (II)
K/R-x^{p}-D/E/N/Q-x^{p}-x₀-S-x₁-x₂-x₃-D/E/N/Q-x^{p}
wherein
x^{p} is a hydrophobic amino acid residue independently selected from A, I, L, V, F or W,
x₀ is K, R or Y, and
x₁, x₂, x₃ are independently any amino acid residue.

38. The peptide according to claim 37, wherein is x₀ K or R.

39. The peptide according to claim 37, wherein x₁, x₂ and x₃ are independently selected from G, V, L, or E.

40. The peptide according to claim 39, wherein x₁ is L or V, x₂ is G and x₃ is E.

41. The peptide according to any of the preceding claims 37-40, wherein said sequence is capable of binding and activating a receptor of the fibroblast growth factor receptor (FGFR) family comprising fibroblast growth factor receptor-1 (FGFR-1), fibroblast growth factor receptor-2 (FGFR-2), fibroblast growth factor receptor-3 (FGFR-3), fibroblast growth factor receptor-4 (FGFR-4) and fibroblast growth factor receptor-5 (FGFR-5).

42. The peptide according to claim 41, wherein the receptor is FGFR-1.

43. The peptide according to claim 42, wherein the sequence is KAEWKSLGEEAWHSK (SEQ ID NO: 1), or a fragment, or a variant of said sequence.

44. The peptide according to any of the preceding claims 37 to 43, wherein said peptide sequence is capable of stimulating neuronal differentiation, neuronal survival and/or neuronal plasticity.

45. The peptide according to claim 44, wherein the neuronal differentiation is differentiation of neural precursor cells and/or differentiation of neurons, such as neurite outgrowth and/or branching the processes.

46. The peptide according to clam 43, wherein the fragment is a peptide sequence of at least three contiguous amino acid residues of the sequence SEQ ID NO: 1 capable of at least one functional activity of the sequence SEQ ID NO: 1 as defined in claims 41, 44 and 45.

47. The peptide according to clam 43, wherein the variant is a peptide sequence which has at least 60 % identity to the sequence of SEQ ID NO: 1 and which is capable of at least one functional activity of the sequence of SEQ ID NO: 1 as said functional activity defined in claims 41, 44 and 45.

48. The isolated peptide according to any of the preceding claims 37 to 47, wherein said peptide is capable of stimulating learning and/or memory

49. A peptide according to any of the preceding claims 37 to 48 for use as a medicament.

50. The peptide according to claim 49, wherein the medicament is for treatment of a condition or disease wherein stimulating neural cell differentiation, neural cell survival, learning and memory, and/or modulating activity of a receptor of the FGFR family is beneficial for said treatment.

51. The peptide according to claim 50, wherein the condition or disease is a condition or disease of the central and peripheral nervous system.

52. The peptide according to claim 51, wherein the condition or disease is selected from postoperative nerve damage, traumatic nerve damage, impaired myelination of nerve fibers, postischaemic damage, such as after stroke, nerve degeneration associated with diabetes mellitus, disorders affecting the circadian clock or neuro-muscular transmission.

53. The peptide according to claim 49, wherein the medicament is for treatment of a condition or disease selected from of conditions or diseases of the muscles including conditions with impaired function of neuro-muscular connections, such as after organ transplantation, or such as genetic or traumatic atrophic muscle disorders.

54. The peptide according to claim 49, wherein the medicament is for treatment of a condition or disease associated with neoagiogenesis, tissue remodelling and/or increased motility of the cells.

55. The peptide according to claim 54, wherein the disease is cancer.

56. The peptide according to claim 55, wherein the cancer is any cancer involving neoangiogenesis.

57. The peptide according to claim 55, wherein the cancer is a cancer of neural system.

58. The peptide according to claim 51, wherein the condition or disease is an impaired ability to learn and/or impaired memory.

59. The peptide according to claim 51 or 58, wherein the condition or disease is Parkinson's disease, Alzheimer's disease, Huntington's disease or dementia such as multiinfarct dementia.

60. The peptide according to claim 51, wherein the condition or disease is a mental disease, such as a disorder of thought and/or mood, neuropsychiatric disorders including bipolar (BPD), genetically related unipolar affective disorders, delusional disorders, paraphrenia, paranoid psychosis, schizophrenia, schizotypal disorder, schizoaffective disorder, schizoaffective bipolar and genetically related unipolar affective disorders, psychogenic psychosis, catatonia, periodic bipolar and genetically related unipolar affective disorders, cycloid psychosis, schizoid personality disorder, paranoid personality disorder, bipolar and genetically related unipolar affective disorders related affective disorders and subtypes of unipolar affective disorder.

61. The peptide according to claim 51, wherein the medicament is for treatment of a condition or disease associated with body damages due to alcohol consumption.

62. The peptide according to claim 51, wherein the medicament is for treatment of prion diseases.

63. A medicament comprising a peptide peptide according to any of the claims 37 to 48.

64. A medicament for stimulating learning and/or memory comprising a peptide according to any of the claims 1 to 18 and/or a peptide according to any of the claims 37 to 48.

65. A pharmaceutical composition comprising an effective amount of a medicament according to claim 63.

66. A pharmaceutical composition comprising an effective amount of a medicament according to claim 64.

67. Use a peptide according to any of the claims 1 to 18 and/or 37 to 48 for the production of an antibody.

68. An antibody capable of recognizing and binding to an epitope comprising a peptide according to any of the claims 1 to 18 and/or 37 to 48.

69. The antibody according to claim 68, wherein said antibody are capable of modulating biological activity mediated by NCAM and/or FGFR.

70. An antibody according to claims 68 and/or 69 for use as a medicament.

71. A pharmaceutical composition comprising an antibody according to claim 68 or 69.

## Patentansprüche

1. Peptid mit 11 bis 18 Aminosäureresten, umfassend das Aminosäuremotiv der Formel (I)
K/R-x^{P}-D/E/N/Q-x^{P}-x₀-S-x₁-x₂-x₃-D/E/N/Q-x^{P}
wobei
x^{P} unabhängig aus A, P, V oder W ausgewählt ist,
x₀ K, R oder Y ist, und
x₁, x₂ und x₃ jeweils unabhängig Aminosäurereste sind.

2. Peptid nach Anspruch 1, wobei x^{P} aus A, V oder W ausgewählt ist.

3. Peptid nach Anspruch 2, wobei das Aminosäuremotiv
K/R-V/A-D/E/N/Q-W-x₀-S-x₁-x₂-x₃-D/E/N/Q-V/A
ist, wobei x₀, x₁, x₂ und x₃ wie in Anspruch 1 definiert sind.

4. Peptid nach Anspruch 1, wobei x^{P} aus A, V oder P ausgewählt ist.

5. Peptid nach Anspruch 4, wobei das Aminosäuremotiv
K/R-V/A-D/E/N/Q-P-x₀-S-x₁-x₂-x₃-D/E/N/Q-V/A
ist, wobei x₀, x₁, x₂ und x₃ wie in Anspruch 1 definiert sind.

6. Peptid nach einem der Ansprüche 1 bis 5, wobei x₁, x₂ und x₃ unabhängig aus A, G, L, S, T oder E ausgewählt sind.

7. Peptid nach Anspruch 6, wobei die Peptidsequenz eine Länge von 11 bis 14 Aminosäureresten aufweist.

8. Peptid nach Anspruch 6, wobei die Peptidsequenz eine Länge von 15 bis 18 Aminosäureresten aufweist.

9. Peptid nach Anspruch 8, wobei die Peptidsequenz KAEWKSLGEEAWHSK (SEQ ID Nr. 1) ist.

10. Peptid nach Anspruch 8, wobei die Peptidsequenz SIDRVNPYSSTAQVQFD (SEQ ID Nr. 3) ist.

11. Peptid nach einem der vorangehenden Ansprüche 1 bis 10, wobei das Peptid als multimere Verbindung formuliert ist, die zwei oder mehrere Kopien der einzelnen Peptidsequenz umfasst.

12. Peptid nach Anspruch 11, wobei die multimere Verbindung ein Dimer ist, umfassend zwei identische Kopien einer einzelnen Peptidsequenz nach den Ansprüchen 1 bis 10.

13. Peptid nach Anspruch 11, wobei die multimere Verbindung ein Dimer ist, umfassend zwei unterschiedliche Peptidsequenzen nach den Ansprüchen 1 bis 10.

14. Peptid nach Anspruch 11, wobei die multimere Verbindung ein Dimer ist, umfassend ein Peptid nach einem der Ansprüche 1 bis 10 und ein Peptid, ausgewählt aus EVYVVAENQQGKSKA (SEQ ID Nr. 4) oder TIMGLKPETRYAVR (SEQ ID Nr. 5).

15. Verfahren nach Anspruch 11, wobei die multimere Verbindung ein Dendrimer ist, umfassend vier identische Kopien eines Peptids nach einem der Ansprüche 1 bis 10, verbunden mit einer Backbone-Struktur von drei Lysinresten.

16. Peptid nach einem der vorstehenden Ansprüche, wobei das Peptidfragment in der Lage ist, einen Rezeptor der Familie der Fibroblasten-Wachstumsfaktor-Rezeptoren (FGFR) zu binden oder zu aktivieren, der Fibroblasten-Wachstumfaktor-Rezeptor-1 (FGFR-1), Fibroblasten-Wachstumfaktor-Rezeptor-2 (FGFR-2), Fibroblasten-Wachstumfaktor-Rezeptor-3 (FGFR-3), Fibroblasten-Wachstumfaktor-Rezeptor-4 (FGFR-4) und Fibroblasten-Wachstumfaktor-Rezeptor-5 (FGFR-5) umfasst.

17. Peptid nach Anspruch 16, wobei der Rezeptor FGFR-1 ist.

18. Peptid nach einem der vorstehenden Ansprüche, wobei das Peptid in der Lage ist, das Lernen und das Gedächtnis zu stimulieren.

19. Peptid mit 11 bis 18 Aminosäureresten, umfassend das Aminosäuremotiv der Formel (I)
K/R-x^{P}-D/E/N/Q-x^{P}-x₀-S-x₁-x₂-x₃-D/E/N/Q-x^{P}
wobei
x^{P} unabhängig A, P, V oder W ist,
x₀ K, R oder Y ist, und
x₁, x₂ und x₃ unabhängig beliebige Aminosäurereste sind,
zur Anwendung bei der Stimulation des Gedächtnisses und des Lernens.

20. Peptid nach Anspruch 19, wobei x^{P} aus A, V oder W ausgewählt ist.

21. Peptid nach Anspruch 20, wobei das Aminosäuremotiv
K/R-V/A-D/E/N/Q-W-x₀-S-x₁-x₂-x₃-D/E/N/Q-V/A
ist, wobei x₀, x₁, x₂ und x₃ wie in Anspruch 1 definiert sind.

22. Peptid nach Anspruch 19, wobei x^{P} aus A, V oder P ausgewählt ist.

23. Peptid nach Anspruch 22, wobei das Aminosäuremotiv
K/R-V/A-D/E/N/Q-P-x₀-S-x₁-x₂-x₃-D/E/N/Q-V/A
ist, wobei x₀, x₁, x₂ und x₃ wie in Anspruch 1 definiert sind.

24. Peptid nach einem der Ansprüche 19 bis 23, wobei x₁, x₂ und x₃ unabhängig aus A, G, L, S, T oder E ausgewählt sind.

25. Peptid nach Anspruch 24, wobei die Peptidsequenz eine Länge von 11 bis 14 Aminosäureresten aufweist.

26. Peptid nach Anspruch 24, wobei die Peptidsequenz eine Länge von 15 bis 18 Aminosäureresten aufweist.

27. Peptid nach Anspruch 26, wobei die Peptidsequenz KAEWKSLGEEAWHSK (SEQ ID Nr. 1) ist.

28. Peptid nach Anspruch 26, wobei die Peptidsequenz SIDRVEPYSSTAQVQFD (SEQ ID Nr. 2) ist.

29. Peptid nach Anspruch 26, wobei die Peptidsequenz SIDRVNPYSSTAQVQFD (SEQ ID Nr. 3) ist.

30. Peptid nach einem der vorstehenden Ansprüche 19 bis 29, wobei eine Peptidsequenz nach einem der Ansprüche 1 bis 18 als multimere Verbindung formuliert ist, umfassend zwei oder mehr Kopien der individuellen Peptidsequenz.

31. Peptid nach Anspruch 30, wobei die multimere Verbindung ein Dimer ist, umfassend zwei identische Kopien einer einzelnen Peptidsequenz nach einem der Ansprüche 1 bis 18.

32. Peptid nach Anspruch 30, wobei die multimere Verbindung ein Dimer ist, umfassend zwei unterschiedliche Peptidsequenzen nach den Ansprüchen 1 bis 18.

33. Peptid nach Anspruch 30, wobei die multimere Verbindung ein Dimer ist, umfassend eine Sequenz nach einem der Ansprüche 1 bis 18 und eine Sequenz, ausgewählt aus TIMGLKPETRYAVR (SEQ ID Nr. 5) oder EVYVVAENQQGKSKA (SEQ ID Nr. 4).

34. Verfahren nach Anspruch 30, wobei die multimere Verbindung ein Dendrimer ist, umfassend vier identische Kopien einer Peptidsequenz nach einem der Ansprüche 1 bis 18, verbunden mit einer Backbone-Struktur von drei Lysinresten.

35. Peptid nach einem der Ansprüche 19 bis 34, wobei das Peptidfragment in der Lage ist, einen Rezeptor der Familie der Fibroblasten-Wachstumsfaktor-Rezeptoren (FGFR) zu binden oder zu aktivieren, der Fibroblasten-Wachstumfaktor-Rezeptor-1 (FGFR-1), Fibroblasten-Wachstumfaktor-Rezeptor-2 (FGFR-2), Fibroblasten-Wachstumfaktor-Rezeptor-3 (FGFR-3), Fibroblasten-Wachstumfaktor-Rezeptor-4 (FGFR-4) und Fibroblasten-Wachstumfaktor-Rezeptor-5 (FGFR-5) umfasst.

36. Peptid nach Anspruch 35, wobei der Rezeptor FGFR-1 ist.

37. Peptid mit 11 bis 18 Aminosäureresten, umfassend die Aminosäuresequenz der Formel (III)
K/R-x^{P}-D/E/N/Q-x^{P}-x₀-S-x₁-x₂-x₃-D/E/N/Q-x^{P}
wobei
x^{P} ein hydrophober Aminosäurerest ist, unabhängig ausgewählt aus A, I, L, V, F oder W,
x₀ K, R oder Y ist, und
x₁, x₂, x₃ unabhängig ein beliebiger Aminosäurerest sind.

38. Peptid nach Anspruch 37, wobei x₀ K oder R ist.

39. Peptid nach Anspruch 37, wobei x₁, x₂ und x₃ unabhängig aus G, V, L oder E gewählt sind.

40. Peptid nach Anspruch 39, wobei x₁ L oder V ist, x₂ G ist und x₃ E ist.

41. Peptid nach einem der vorstehenden Ansprüche 37 bis 40, wobei die Sequenz in der Lage ist, einen Rezeptor der Familie der Fibroblasten-Wachstumsfaktor-Rezeptoren (FGFR) zu binden oder zu aktivieren, der Fibroblasten-Wachstumfaktor-Rezeptor-1 (FGFR-1), Fibroblasten-Wachstumfaktor-Rezeptor-2 (FGFR-2), Fibroblasten-Wachstumfaktor-Rezeptor-3 (FGFR-3), Fibroblasten-Wachstumfaktor-Rezeptor-4 (FGFR-4) und Fibroblasten-Wachstumfaktor-Rezeptor-5 (FGFR-5) umfasst.

42. Peptid nach Anspruch 41, wobei der Rezeptor FGFR-1 ist.

43. Peptid nach Anspruch 42, wobei die Sequenz KAEWKSLGEEAWHSK (SEQ ID Nr. 1) oder ein Fragment oder eine Variante der Sequenz ist.

44. Peptid nach einem der vorstehenden Ansprüche 37 bis 43, wobei die Peptidsequenz in der Lage ist, die neuronale Differenzierung, das neuronale Überleben und/oder die neuronale Plastizität zu stimulieren.

45. Peptid nach Anspruch 44, wobei die neuronale Differenzierung die Differenzierung von neuralen Vorläuferzellen und/oder die Differenzierung von Neuronen wie Neuritenüberwuchs und/oder -verzweigung der Prozesse ist.

46. Peptid nach Anspruch 43, wobei das Fragment eine Peptidsequenz von zumindest drei angrenzenden Aminosäureresten der Sequenz SED Nr. 1 ist, die zu zumindest eine funktionelle Aktivität der Sequenz SEQ ID Nr. 1, wie in den Ansprüchen 41, 44 und 45 definiert, aufweisen kann.

47. Peptid nach Anspruch 43, wobei der Variant eine Peptidsequenz ist, die zumindest 60 % mit der Sequenz von SEQ ID Nr. 1 identisch und zu zumindest eine funktionelle Aktivität der Sequenz von SEQ ID Nr. 1, wie die funktionelle Aktivität nach Anspruch 41,44 und 45, aufweisen kann.

48. Isoliertes Peptid nach einem der vorstehenden Ansprüche 37 bis 47, wobei das Peptid in der Lage ist, das Lernen und/oder das Gedächtnis zu stimulieren.

49. Peptid nach einem der vorstehenden Ansprüche 37 bis 48 zur Verwendung als Medikament.

50. Peptid nach Anspruch 49, wobei das Medikament zur Behandlung eines Zustands oder einer Erkrankung vorgesehen ist, wobei das Stimulieren der neuralen Zelldifferenzierung, des neuralen Zellüberlebens, des Lernens und des Gedächtnisses und/oder das Modulieren der Aktivität eines Rezeptors der FGFR-Familie für die Behandlung vorteilhaft ist bzw. sind.

51. Peptid nach Anspruch 50, wobei der Zustand oder die Erkrankung ein Zustand oder eine Erkrankung des zentralen und peripheren Nervensystems ist.

52. Peptid nach Anspruch 51, wobei das Leiden oder die Erkrankung aus postoperativem Nervenschaden, traumatischem Nervenschaden, beeinträchtigter Myelinisierung von Nervenfasern, postischämischem Schaden, wie nach einem Schlaganfall, Nervendegeneration, die mit Diabetes mellitus assoziiert ist, Störungen, die die zirkadiane Uhr oder die neuromuskuläre Übertragung betreffen., ausgewählt ist.

53. Peptid nach Anspruch 49, wobei das Medikament zur Behandlung eines Zustands oder einer Erkrankung vorgesehen ist, ausgewählt aus Zuständen oder Erkrankungen der Muskeln, einschließlich Zuständen mit beeinträchtigter Funktion der neuromuskulären Verbindungen wie schlaganfallbedingte Organtransplantation oder beispielsweise genetische oder traumatische atrophische Muskelstörungen.

54. Peptid nach Anspruch 49, wobei das Medikament zur Behandlung eines Zustands oder einer Erkrankung vorgesehen ist, der bzw. die mit Neoangiogenese, Geweberemodellierung und/oder erhöhter Motilität der Zellen assoziiert ist.

55. Peptid nach Anspruch 54, wobei die Erkrankung Krebs ist.

56. Peptid nach Anspruch 55, wobei der Krebs ein Krebs ist, der Neoangiogenese umfasst.

57. Peptid nach Anspruch 55, wobei der Krebs ein Krebs des Nervensystems ist.

58. Peptid nach Anspruch 51, wobei der Zustand oder die Erkrankung eine beeinträchtigte Lernfähigkeit und/oder ein beeinträchtigtes Gedächtnis ist.

59. Peptid nach Anspruch 51 oder 58, wobei der Zustand oder die Erkrankung Morbus Parkinson, Morbus Alzheimer, Morbus Huntington oder Demenz wie Multiinfarktdemenz ist.

60. Peptid nach Anspruch 51, wobei der Zustand oder die Erkrankung eine geistige Erkrankung ist, beispielsweise eine Denk- und/oder Befindlichkeitsstörung, neuropsychiatrische Störungen, beispielsweise bipolare (BPD), genetisch bedingte unipolare affektive Störungen, wahnhafte Störungen, Paraphrenie, paranoide Psychose, Schizophrenie, schizotype Störung, schizoaffektive Störung, schizoaffektive bipolare und genetisch bedingte unipolare affektive Störungen, psychogene Psychose, Katatonie, periodische bipolare und genetisch bedingte unipolare affektive Störungen, zykloide Psychose, schizoide Persönlichkeitsstörung, paranoide Persönlichkeitsstörung, bipolare und genetisch bedingte unipolare affektive Störungen, verwandte affektive Störungen und Subtypen der unipolaren affektiven Störung.

61. Peptid nach Anspruch 51, wobei das Medikament zur Behandlung eines Zustands oder einer Erkrankung vorgesehen ist, der bzw. die mit durch Alkoholkonsum hervorgerufenen Körperschäden assoziiert ist.

62. Peptid nach Anspruch 51, wobei das Medikament zur Behandlung von Prionerkrankungen vorgesehen ist.

63. Medikament, umfassend ein Peptid nach einem der Ansprüche 37 bis 48.

64. Medikament zum Stimulieren des Lernens und/oder des Gedächtnisses, umfassend ein Peptid nach einem der Ansprüche 1 bis 18 und/oder ein Peptid nach einem der Ansprüche 37 bis 48.

65. Pharmazeutische Zusammensetzung, umfassend eine effektive Menge eines Medikaments nach Anspruch 63.

66. Pharmazeutische Zusammensetzung, umfassend eine effektive Menge eines Medikaments nach Anspruch 64.

67. Verwendung eines Peptids nach einem der Ansprüche 1 bis 18 und/oder 37 bis 48 zur Herstellung eines Antikörpers.

68. Antikörper, der in der Lage ist, ein Epitop zu erkennen und an dieses zu binden, umfassend ein Peptid nach einem der Ansprüche 1 bis 18 und/oder 37 bis 48.

69. Antikörper nach Anspruch 68, wobei der Antikörper in der Lage ist, eine biologische Aktivität, die von NCAM und/oder FGFR vermittelt wird, zu modulieren.

70. Antikörper nach den Ansprüchen 68 und/oder 69 zur Verwendung als Medikament.

71. Pharmazeutische Zusammensetzung, umfassend einen Antikörper nach Anspruch 68 oder 69.

## Revendications

1. Peptide de 11 à 18 résidus d'acides aminés comprenant le motif d'acides aminés de la formule (I)
K/R-x^{P}-D/E/N/Q-x^{P}-x₀-S-x₁-x₂-x₃-D/E/N/Q-x^{P}
dans laquelle
x^{P} est indépendamment sélectionné parmi A, P, V ou W,
x₀ est K, R ou Y, et
x₁, x₂ et x₃ sont indépendamment un quelconque résidu d'acide aminé.

2. Peptide selon la revendication 1, dans lequel x^{P} est sélectionné parmi A, V ou W.

3. Peptide selon la revendication 2, dans lequel le motif d'acides aminés est
K/R-V/A-D/E/N/Q-W-x₀-S-x₁-x₂-x₃-D/E/N/Q-V/A,
où x₀, x₁, x₂ et x₃ sont tels que définis dans la revendication 1.

4. Peptide selon la revendication 1, dans lequel x^{P} est sélectionné parmi A, V ou P.

5. Peptide selon la revendication 4, dans lequel le motif d'acides aminés est K/R-V/A-D/E/N/Q-P-x₀-S-x₁-x₂-x₃-D/E/N/Q-V/A,
où x₀, x₁, x₂ et x₃ sont tels que définis dans la revendication 1.

6. Peptide selon l'une quelconque des revendications 1 à 5, dans lequel x₁, x₂ et x₃ sont indépendamment sélectionnés parmi A, G, L, S, T, ou E.

7. Peptide selon la revendication 6, dans lequel la séquence peptidique possède une longueur de 11 à 14 résidus d'acides aminés.

8. Peptide selon la revendication 6, dans lequel la séquence peptidique possède une longueur de 15 à 18 résidus d'acides aminés.

9. Peptide selon la revendication 8, dans lequel la séquence peptidique est KAEWKSLGEEAWHSK (SEQ ID NO: 1).

10. Peptide selon la revendication 8, dans lequel la séquence peptidique est SIDRVNPYSSTAQVQFD (SEQ ID NO: 3).

11. Peptide selon l'une quelconque des revendications 1 à 10 précédentes, où ledit peptide est formulé en tant que composé multimérique comprenant deux copies ou davantage de ladite séquence peptidique individuelle.

12. Peptide selon la revendication 11, où le composé multimérique est un dimère comprenant deux copies identiques d'une séquence peptidique individuelle selon l'une quelconque des revendications 1 à 10.

13. Peptide selon la revendication 11, dans lequel le composé multimérique est un dimère comprenant deux séquences peptidiques différentes selon les revendications 1 à 10.

14. Peptide selon la revendication 11, dans lequel le composé multimérique est un dimère comprenant un peptide selon l'une quelconque des revendications 1 à 10 et un peptide sélectionné parmi EVYVVAENQQGKSKA (SEQ ID NO: 4) ou TIMGLKPETRYAVR (SEQ ID NO: 5).

15. Peptide selon la revendication 11, dans lequel le composé multimérique est un dendrimère comprenant quatre copies identiques d'un peptide selon l'une quelconque des revendications 1 à 10, liées à une structure de squelette de trois résidus lysine.

16. Peptide selon l'une quelconque des revendications précédentes, dans lequel le fragment peptidique est capable de se lier à et d'activer un récepteur de la famille des récepteurs du facteur de croissance des fibroblastes (FGFR) comprenant le récepteur 1 du facteur de croissance des fibroblastes (FGFR-1), le récepteur 2 du facteur de croissance des fibroblastes (FGFR-2), le récepteur 3 du facteur de croissance des fibroblastes (FGFR-3), le récepteur 4 du facteur de croissance des fibroblastes (FGFR-4) et le récepteur 5 du facteur de croissance des fibroblastes (FGFR-5).

17. Peptide selon la revendication 16, où le récepteur est le FGFR-1.

18. Peptide selon l'une quelconque des revendications précédentes, ledit peptide étant capable de stimuler l'apprentissage et la mémoire.

19. Peptide de 11 à 18 résidus d'acides aminés comprenant le motif d'acides aminés de la formule (I)
K/R-x^{P}-D/E/N/Q-x^{P}-x₀-S-x₁-x₂-x₃-D/E/N/Q-x^{P}
dans laquelle
x^{P} est indépendamment A, P, V ou W,
x₀ est K, R ou Y, et
x₁, x₂ et x₃ sont indépendamment un quelconque résidu d'acide aminé,
destiné à être utilisé pour stimuler la mémoire et l'apprentissage.

20. Peptide selon la revendication 19, dans lequel x^{P} est sélectionné parmi A, V ou W.

21. Peptide selon la revendication 20, dans lequel le motif d'acides aminés est
K/R-V/A-D/E/N/Q-W-x₀-S-x₁-x₂-x₃-D/E/N/Q-V/A,
où x₀, x₁, x₂ et x₃ sont tels que définis dans la revendication 1.

22. Peptide selon la revendication 19, dans lequel x^{P} est sélectionné parmi A, V ou P.

23. Peptide selon la revendication 22, dans lequel le motif d'acides aminés est
K/R-V/A-D/E/N/Q-P-x₀-S-x₁-x₂-x₃-D/E/N/Q-V/A,
où x₀, x₁, x₂ et x₃ sont tels que définis dans la revendication 1.

24. Peptide selon l'une quelconque des revendications 19 à 23, dans lequel x₁, x₂ et x₃ sont indépendamment sélectionnés parmi A, G, L, S, T, ou E.

25. Peptide selon la revendication 24, dans lequel la séquence peptidique possède une longueur de 11 à 14 résidus d'acides aminés.

26. Peptide selon la revendication 24, dans lequel la séquence peptidique possède une longueur de 15 à 18 résidus d'acides aminés.

27. Peptide selon la revendication 26, dans lequel la séquence peptidique est KAEWKSLGEEAWHSK (SEQ ID NO: 1).

28. Peptide selon la revendication 26, dans lequel la séquence peptidique est SIDRVEPYSSTAQVQFD (SEQ ID NO: 2).

29. Peptide selon la revendication 26, dans lequel la séquence peptidique est SIDRVNPYSSTAQVQFD (SEQ ID NO: 3).

30. Peptide selon l'une quelconque des revendications 19 à 29 précédentes, où une séquence peptidique selon l'une quelconque des revendications 1 à 18 est formulée en tant que composé multimérique comprenant deux copies ou davantage de ladite séquence peptidique individuelle.

31. Peptide selon la revendication 30, où le composé multimérique est un dimère comprenant deux copies identiques d'une séquence peptidique individuelle selon l'une quelconque des revendications 1 à 18.

32. Peptide selon la revendication 30, dans lequel le composé multimérique est un dimère comprenant deux séquences peptidiques différentes selon les revendications 1 à 18.

33. Peptide selon la revendication 30, dans lequel le composé multimérique est un dimère comprenant une séquence selon l'une quelconque des revendications 1 à 18 et une séquence sélectionnée parmi TIMGLKPETRYAVR (SEQ ID NO: 5) ou EVYVVAENQQGKSKA (SEQ ID NO: 4).

34. Peptide selon la revendication 30, dans lequel le composé multimérique est un dendrimère comprenant quatre copies identiques d'une séquence peptidique selon l'une quelconque des revendications 1 à 18, liées à une structure de squelette de trois résidus lysine.

35. Peptide selon l'une quelconque des revendications 19 à 34 précédentes, dans lequel le fragment peptidique est capable de se lier à et d'activer un récepteur de la famille des récepteurs du facteur de croissance des fibroblastes (FGFR) comprenant le récepteur 1 du facteur de croissance des fibroblastes (FGFR-1), le récepteur 2 du facteur de croissance des fibroblastes (FGFR-2), le récepteur 3 du facteur de croissance des fibroblastes (FGFR-3), le récepteur 4 du facteur de croissance des fibroblastes (FGFR-4) et le récepteur 5 du facteur de croissance des fibroblastes (FGFR-5).

36. Peptide selon la revendication 35, où le récepteur est le FGFR-1.

37. Peptide de 11 à 18 résidus d'acides aminés comprenant la séquence d'acides aminés de la formule (II)
K/R-x^{P}-D/E/N/Q-x^{P}-x₀-S-x₁-x₂-x₃-D/E/N/Q-x^{P}
dans laquelle
x^{P} est un résidu d'acide aminé hydrophobe indépendamment sélectionné parmi A, I, L, V, F ou W,
x₀ est K, R ou Y, et
x₁, x₂ et x₃ sont indépendamment un quelconque résidu d'acide aminé.

38. Peptide selon la revendication 37, dans lequel x₀ est K ou R.

39. Peptide selon la revendication 37, dans lequel x₁, x₂ et x₃ indépendamment sélectionnés parmi G, V, L, ou E.

40. Peptide selon la revendication 39, dans lequel x₁ est L ou V, x₂ est G et x₃ est E.

41. Peptide selon l'une quelconque des revendications 37 à 40 précédentes, dans lequel ladite séquence est capable de se lier à et d'activer un récepteur de la famille des récepteurs du facteur de croissance des fibroblastes (FGFR) comprenant le récepteur 1 du facteur de croissance des fibroblastes (FGFR-1), le récepteur 2 du facteur de croissance des fibroblastes (FGFR-2), le récepteur 3 du facteur de croissance des fibroblastes (FGFR-3), le récepteur 4 du facteur de croissance des fibroblastes (FGFR-4) et le récepteur 5 du facteur de croissance des fibroblastes (FGFR-5).

42. Peptide selon la revendication 41, dans lequel le récepteur est le FGFR-1.

43. Peptide selon la revendication 42, dans lequel la séquence est KAEWKSLGEEAWHSK (SEQ ID NO: 1), ou un fragment ou un variant de ladite séquence.

44. Peptide selon l'une quelconque des revendications 37 à 43 précédentes, dans lequel ladite séquence peptidique est capable de stimuler la différenciation neuronale, la survie neuronale et/ou la plasticité neuronale.

45. Peptide selon la revendication 44, où la différenciation neuronale est la différenciation de cellules précurseurs neurales et/ou la différenciation de neurones, comme l'excroissance des neurites et/ou la ramification des prolongements.

46. Peptide selon la revendication 43, dans lequel le fragment est une séquence peptidique d'au moins trois résidus d'acides aminés contigus de la séquence SEQ ID NO: 1, qui est capable de présenter au moins une activité fonctionnelle de la séquence SEQ ID NO: 1 telle que définie dans les revendications 41, 44 et 45.

47. Peptide selon la revendication 43, dans lequel le variant est une séquence peptidique qui possède au moins 60 % d'identité avec la séquence SEQ ID NO: 1 et qui est capable de présenter au moins une activité fonctionnelle de la séquence SEQ ID NO: 1, comme ladite activité fonctionnelle définie dans les revendications 41, 44 et 45.

48. Peptide isolé selon l'une quelconque des revendications 37 à 47 précédentes, où ledit peptide est capable de stimuler l'apprentissage et/ou la mémoire.

49. Peptide selon l'une quelconque des revendications 37 à 48 précédentes, destiné à être utilisé en tant que médicament.

50. Peptide selon la revendication 49, où le médicament est destiné au traitement d'un état pathologique ou d'une maladie dans lequel/laquelle une stimulation de la différenciation des cellules neurales, de la survie des cellules neurales, de l'apprentissage et de la mémoire, et/ou une modulation de l'activité d'un récepteur de la famille FGFR est bénéfique pour ledit traitement.

51. Peptide selon la revendication 50, où l'état pathologique ou la maladie est un état pathologique ou une maladie du système nerveux central et périphérique.

52. Peptide selon la revendication 51, où l'état pathologique ou la maladie est sélectionné(e) parmi des lésions nerveuses postopératoires, des lésions nerveuses traumatiques, une myélinisation des fibres nerveuses altérée, des lésions post-ischémiques comme après un AVC, une dégénérescence nerveuse associée au diabète sucré, des troubles affectant l'horloge circadienne ou la transmission neuromusculaire.

53. Peptide selon la revendication 49, où le médicament est destiné au traitement d'un état pathologique ou d'une maladie sélectionné(e) parmi des états pathologiques ou des maladies des muscles comprenant des états pathologiques impliquant une fonction des connexions neuromusculaires altérée, comme après une greffe d'organe, ou comme des troubles musculaires atrophiques génétiques ou traumatiques.

54. Peptide selon la revendication 49, où le médicament est destiné au traitement d'un état pathologique ou d'une maladie associé(e) à la néoangiogenèse, au remodelage tissulaire et/ou à une motilité des cellules augmentée.

55. Peptide selon la revendication 54, où la maladie est un cancer.

56. Peptide selon la revendication 55, où le cancer est un cancer quelconque impliquant une néoangiogenèse.

57. Peptide selon la revendication 55, où le cancer est un cancer du système neural.

58. Peptide selon la revendication 5 1 , o ù l' é t a t pathologique ou la maladie est un trouble de l'apprentissage et/ou un trouble de la mémoire.

59. Peptide selon la revendication 51 ou 58, où l'état pathologique ou la maladie est la maladie de Parkinson, la maladie d'Alzheimer, la maladie de Huntington ou une démence comme la démence par infarctus multiples.

60. Peptide selon la revendication 51, où l'état pathologique ou la maladie est une maladie mentale, comme un trouble de la pensée et/ou de l'humeur, des troubles neuropsychiatriques comprenant des troubles bipolaires (BPD), des troubles affectifs unipolaires d'origine génétique, des troubles délirants, la paraphrénie, la psychose paranoïaque, la schizophrénie, un trouble schizotypique, un trouble schizo-affectif, des troubles bipolaires schizo-affectifs et des troubles affectifs unipolaires d'origine génétique, une psychose psychogène, la catatonie, des troubles affectifs unipolaires d'origine génétique et bipolaires périodiques, une psychose cycloïde, un trouble de la personnalité schizoïde, un trouble de la personnalité paranoïaque, des troubles affectifs apparentés à des troubles affectifs unipolaires d'origine génétique et bipolaires, et des sous-types de trouble affectif unipolaire.

61. Peptide selon la revendication 51, où le médicament est destiné au traitement d'un état pathologique ou d'une maladie associé(e) à des lésions corporelles dues à la consommation d'alcool.

62. Peptide selon la revendication 51, où le médicament est destiné au traitement de maladies à prion.

63. Médicament comprenant un peptide selon l'une quelconque des revendications 37 à 48.

64. Médicament pour stimuler l'apprentissage et/ou la mémoire comprenant un peptide selon l'une quelconque des revendications 1 à 18 et/ou un peptide selon l'une quelconque des revendications 37 à 48.

65. Composition pharmaceutique comprenant une quantité efficace d'un médicament selon la revendication 63.

66. Composition pharmaceutique comprenant une quantité efficace d'un médicament selon la revendication 64.

67. Utilisation d'un peptide selon l'une quelconque des revendications 1 à 18 et/ou 37 à 48 pour la production d'un anticorps.

68. Anticorps capable de reconnaître et de se lier à un épitope comprenant un peptide selon l'une quelconque des revendications 1 à 18 et/ou 37 à 48.

69. Anticorps selon la revendication 68, où ledit anticorps est capable de moduler une activité biologique médiée par la NCAM et/ou le FGFR.

70. Anticorps selon les revendications 68 et/ou 69, destiné à être utilisé en tant que médicament.

71. Composition pharmaceutique comprenant un anticorps selon la revendication 68 ou 69.


### SEQUENCE LISTING

<110> ENKAM Pharmaceuticals A/S
<120> FGFR binding peptides
<130> P955PC00
<160> 5
<170> Patent In version 3.5
<210> 1
   <211> 15
   <212> PRT
   <213> Artificial sequence
<220>
   <223> CDL peptide, fragment of NCAM F3,1
<400> 1
   Lys Ala Glu Trp Lys Ser Leu Gly Glu Glu Ala Trp His Ser Lys
   1               5                   10                  15
<210> 2
   <211> 17
   <212> PRT
   <213> Artificial sequence
<220>
   <223> ABL peptide, fragment of NCAM F3,1
<400> 2
   Ser Ile Asp Arg Val Glu Pro Tyr Ser Ser Thr Ala Gln Val Gln Phe
   1 5 10 15
   Asp
<210> 3
   <211> 17
   <212> PRT
   <213> Artificial sequence
<220>
   <223> E/N mutant of ABL peptide
<400> 3
   Ser Ile Asp Arg Val Asn Pro Tyr Ser Ser Thr Ala Gln Val Gln Phe
   1 5 10 15
   Asp
<210> 4
   <211> 15
   <212> PRT
   <213> Artificial Sequence
<220>
   <223> FGL peptide, fragment of NCAM F3,2
<400> 4
   Glu Val Tyr Val Val Ala Glu Asn Gln Gln Gly Lys Ser Lys Ala
   1 5 10 15
<210> 5
   <211> 14
   <212> PRT
   <213> Artificial sequence
<220>
   <223> EFL peptide, fragment of NCAM F3,1
<400> 5
   Thr Ile Met Gly Leu Lys Pro Glu Thr Arg Tyr Ala Val Arg
   1 5 10